# EUROPEAN PATENT APPLICATION

(11) **EP 4 852 003 A1**
(43) Date of publication of application: **09.12.2026**
(21) Application number: 26182758.8
(22) Date of filing: 02.06.2026
(51) Int. Cl.: G16B 40/20, G16B 25/10, G16H 20/10, G16H 50/20

(54) **SYSTEM AND METHOD FOR PREDICTING MOLECULAR RELAPSE IN CHRONIC MYELOID LEUKEMIA PATIENTS USING MACHINE LEARNING**

(30) Priority: 02.06.2025 KR 20250072006
(71) Applicant: Eulji University Industry Academy Cooperation Foundation, Seongnam-si, Gyeonggi-do 13135 (KR); OmicSyn Co., Ltd., Seoul 05644 (KR)
(72) Inventor: KIM, Dong-Wook, 07811 Seoul (KR)
(74) Representative: Dehns

(57) **Abstract**

The present invention provides a novel machine learning-based computer-implemented system, method, and composition for predicting the likelihood of recurrence in chronic myeloid leukemia, which can rapidly and accurately predict the likelihood of recurrence after discontinuation of a tyrosine kinase inhibitor (TKI) through transcriptome analysis of biological samples of patients and machine learning algorithm.

## Description

### CROSS-REFERENCE TO RELATED APPLICATIONS

This application claims priority to and the benefit of Korean Patent Application No. 10-2025-0072006, filed on June 2, 2025, the disclosures of which are incorporated herein by reference in their entireties.

### TECHNICAL FIELD

The present invention relates to system and method for predicting molecular relapse in chronic myeloid leukemia patients using machine learning system.

### BACKGROUND ART

Chronic Myeloid Leukemia (CML) is a hematological disease characterized by increased proliferation of undifferentiated cells due to the formation of the BCR-ABL fusion protein, which functions as a constitutively active kinase (Savona M, et al., Nat. Rev. Cancer. 8(5):341-350. 2008). The BCR-ABL1 fusion protein is formed by a reciprocal translocation between chromosomes 9 and 22, generating the so-called Philadelphia chromosome, which induces the proliferation of BCR-ABL1-expressing leukemic cells in CML patients. Tyrosine Kinase Inhibitors (TKIs) targeting BCR-ABL1 have remarkably improved clinical outcomes in CML (O'Hare, T et al., Nat. Rev. Cancer 12(8): 513-526. 2012). For instance, the estimated 10-year overall survival rate of chronic-phase CML patients treated with imatinib, a first-generation TKI, was demonstrated to be 83.3% in the International Randomized Study of Interferon and STI571 trial.

Accordingly, the current objective of CML patient management is to achieve Treatment-Free Remission (TFR) while reducing medical expenses without causing severe adverse events after TKI discontinuation. However, recent studies indicate that the successful TFR rate is approximately 40-65%, demonstrating that nearly half of CML patients experience molecular relapse following TKI cessation. A longer duration of TKI therapy or a prolonged molecular response prior to TKI discontinuation has been significantly associated with a reduced risk of losing a Major Molecular Response (MMR).

In addition to clinical features, various biomarkers have been revealed to correlate with TFR. Several studies have reported that an increased proportion or count of cytotoxic CD56^{dim} Natural Killer (NK) cells in peripheral blood at the time of TKI discontinuation serves as a prognostic factor for molecular relapse-free survival (Rea, D. et al., Haematologica 102(8): 1368-1377. 2017). Furthermore, other clinical trials have shown that CML patients with elevated CD86⁺ pDC levels in peripheral blood immediately prior to TKI cessation exhibit a greater risk of relapse compared to patients with lower CD86⁺ pDC levels. Despite these clinical and biological factors associated with successful treatment cessation, the necessity to identify biological factors within the bone marrow (BM) of CML patients to predict the risk of relapse after TKI discontinuation has remained unfulfilled to date (Stuckey, R. et al., Curr. Oncol. Rep. 24(4): 415-426, 2022).

### DETAILED DESCRIPTION OF THE INVENTION

### TECHNICAL PROBLEM

However, investigation into the characterization of heterogeneous cellular compositions within bone marrow samples from Chronic Myeloid Leukemia (CML) patients and their specific correlation with Treatment-Free Remission (TFR) remains an unchartered territory.

The present invention has been made to address the aforementioned and various other problems, and an object of the present invention is to provide a machine learning-based system and method capable of rapidly and accurately predicting the probability of molecular relapse following the discontinuation of a Tyrosine Kinase Inhibitor (TKI), as well as outputting optimized prescriptive recommendations for appropriate therapeutic agents, via transcriptomic analysis of hematological malignancies.

These stated objects, however, are merely exemplary, and the scope of the present invention is not limited thereby.

### TECHNICAL SOLUTION

In an aspect of the present invention, there is provided a computer-implemented system for predicting molecular relapse in a patient having chronic myeloid leukemia (CML) undergoing a tyrosine kinase inhibitor (TKI) discontinuation protocol, the system comprising:
a biomarker data input interface configured to receive an immunological data vector generated from a biological sample of a CML patient at a TKI discontinuation milestone or a subsequent relapse monitoring milestone, wherein said data vector comprises a measured proportion of a first exhausted T-cell (Tₑₓ) subpopulation characterized as GZMK⁺ IL7R⁻ Tₑₓ cells and a measured proportion of a second Tₑₓ subpopulation characterized as GZMK⁺ IL7R⁺ Tₑₓ cells, each relative to total CD8⁺ T cells isolated from the biological sample;
a machine learning pipeline processor communicatively coupled to the interface and containing a trained machine learning classification network built on a machine learning algorithm, wherein the pipeline processor is configured to execute automated processing of the data vector by evaluating a numerical equilibrium between the proportion of the GZMK⁺ IL7R⁻ Tₑₓ cells and the proportion of the GZMK⁺ IL7R⁺ Tₑₓ cells to calculate a long-term molecular relapse probability score and classify the patient into either a high molecular relapse risk state or a stable treatment-free remission (TFR) state; and
a therapeutic intervention interface configured to generate and electronically display an interactive clinical dashboard partitioning the patient management profile into a target clinical roadmap according to the classification outputted by the pipeline processor,
wherein the dashboard outputs a specific digital instruction that directs a clinical practitioner to maintain the TKI discontinuation protocol without drug administration when the patient is classified into the stable TFR state, or to execute a predictive frontline TKI resumption regimen when the patient is classified into the high molecular relapse risk state.

In another aspect of the present invention, there is provided a computer-implemented method for predicting a probability of molecular relapse in a chronic myeloid leukemia (CML) patient at a tyrosine kinase inhibitor (TKI) discontinuation milestone, the method comprising:
compiling, via a digital data input interface, an immunological data vector generated from a bone marrow or peripheral blood sample obtained from the CML patient at the TKI discontinuation milestone, wherein the data vector comprises a measured proportion of a first exhausted T-cell (Tₑₓ) subpopulation characterized as GZMK⁺ IL7R⁻ Tₑₓ cells and a measured proportion of a second Tₑₓ subpopulation characterized as GZMK⁺ IL7R⁺ Tₑₓ cells, each quantified relative to total CD8⁺ T cells isolated from the sample; routing the immunological data vector into a trained machine learning classification network executed by a computing processor, the classification network built on a binary Random Forest algorithm;
calculating, via the computing processor, a molecular relapse probability score by executing automated processing of the data vector through the binary Random Forest algorithm to evaluate a non-linear numerical equilibrium between the proportion of the GZMK⁺ IL7R⁻ Tₑₓ cells and the proportion of the GZMK⁺ IL7R⁺ Tₑₓ cells; and
generating, via a data output interface, a digital prognostic risk profile classifying the patient into either a high molecular relapse risk state or a stable treatment-free remission (TFR) state based on the calculated molecular relapse probability score, thereby providing an analytical indicator for assessing treatment-free remission feasibility prior to drug cessation.

In another aspect of the present invention, there is provided a method for treating a patient having chronic myeloid leukemia (CML) undergoing a tyrosine kinase inhibitor (TKI) discontinuation protocol, the method comprising:
measuring a specific biomarker combination in a bone marrow or peripheral blood sample obtained from the CML patient at a TKI discontinuation milestone, the biomarker combination comprising a proportion of GZMK⁺ IL7R⁻ Tₑₓ cells and a proportion of GZMK⁺ IL7R⁺ Tₑₓ cells relative to total CD8⁺ T cells;
determining a molecular relapse risk state of the patient based on the measured specific biomarker combination, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
modifying the patient's therapeutic regimen according to the determined molecular relapse risk state, by either:
   (i) resuming or continuing a TKI administration protocol or switching the TKI to a 3rd or 4th generation TKI or providing combination therapy of an adjuvant or an immune check point inhibitor with the TKI when the patient is determined to be in the high molecular relapse risk state, or
   (ii) maintaining the TKI discontinuation protocol when the patient is determined to be in the stable TFR state.

In another aspect of the present invention, there is provided a composition for detecting a specific biomarker combination in a bone marrow or peripheral blood samples obtained from chronic myeloid leukemia (CML) patient undergoing a tyrosine kinase inhibitor (TKI) discontinuation protocol comprising reagents for detecting the specific biomarker combination, wherein the specific biomarker combination comprises a proportion of GZMK⁺ IL7R⁻ Tₑₓ cells and a proportion of GZMK⁺ IL7R⁺ Tₑₓ cells relative to total CD8⁺ T cells, for use in a method of managing a CML patient to predict molecular relapse after TKI discontinuation, wherein the method comprises:
measuring said proportion of GZMK⁺ IL7R⁻ Tₑₓ cells and said proportion of GZMK⁺ IL7R⁺ Tₑₓ cells in the biological sample obtained from the patient;
determining a molecular relapse risk state of the patient based on the measured specific biomarker combination, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
identifying a therapeutic intervention for the patient, wherein a frontline TKI administration protocol is resumed or the prescription is switched to a 3rd or 4th generation TKI or combination therapy of an adjuvant or an immune check point inhibitor with the TKI (the frontline TKI, or the 3^{rd} or 4^{th} generation TKI, preferably the frontline TKI) when the patient is determined to be in the high molecular relapse risk state, and the TKI discontinuation protocol is maintained without drug administration when the patient is determined to be in the stable TFR state.

In another aspect, the invention provides an *ex vivo* method of managing a chronic myeloid leukemia (CML) patient to predict molecular relapse after tyrosine kinase inhibitor (TKI) discontinuation (or an *ex vivo* method of predicting molecular relapse of a CML patient after TKI discontinuation and identifying a therapeutic intervention for said patient, or an *ex vivo* method of identifying a therapeutic intervention for a CML patient undergoing a TKI discontinuation protocol), wherein the method comprises:
measuring the proportion of GZMK⁺ IL7R⁻ exhausted T (Tₑₓ) cells and the proportion of GZMK⁺ IL7R⁺ Tₑₓ cells relative to total CD8⁺ T cells in a biological sample (e.g. a bone marrow or peripheral blood sample) that has been obtained from a CML patient undergoing tyrosine kinase inhibitor (TKI) discontinuation (undergoing a TKI discontinuation protocol);
determining a molecular relapse risk state of the patient based on said measured proportions, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
identifying a therapeutic intervention for the patient, wherein a frontline TKI administration protocol is selected for resumption (or alternatively viewed, a decision is made to resume a frontline TKI administration protocol) or the prescription is switched to a 3^{rd} or 4^{th} generation TKI or combination therapy of an adjuvant or an immune check point inhibitor with the TKI (the frontline TKI, or the 3^{rd} or 4^{th} generation TKI, preferably the frontline TKI) when the patient is determined to be in the high molecular relapse risk state, and/or the TKI discontinuation (TKI discontinuation protocol) is maintained without drug administration when the patient is determined to be in the stable TFR state.

Discussion of various features of other aspects and embodiments of the invention may be applied, *mutatis mutandis,* to this aspect of the invention.

### ADVANTAGEOUS EFFECTS

The system and method for predicting the probability of molecular relapse in hematological malignancies according to the present invention, configured as described above, accurately predicts the probability of relapse after the discontinuation of a tyrosine kinase inhibitor (TKI).

Accordingly, the present marker can identify patients at high risk of relapse to recommend the continued use of a TKI or provide additional therapeutic options. Furthermore, the present invention can contribute to improving patient prognosis and preventing disease recurrence by facilitating the development of novel therapeutic modalities targeting leukemic granulocyte-monocyte progenitors (GMPs). However, the scope of the present invention is not limited thereto.

### BRIEF DESCRIPTION OF DRAWINGS

FIG. 1A is a schematic diagram illustrating an experimental design process for longitudinal single-cell RNA sequencing (scRNA-seq) profiling of chronic myeloid leukemia (CML) with relapse or non-relapse after tyrosine kinase inhibitor (TKI) discontinuation, depicting the number of patients (n) and sampling time points.
FIG. 1B is a graph illustrating the results of Uniform Manifold Approximation and Projection (UMAP) visualization analysis of major cell types in longitudinal scRNA-seq profiling of CML with relapse or non-relapse after TKI discontinuation, wherein each dot represents a single cell and is color-coded by cell type.
FIG. 1C is a graph illustrating the results of UMAP visualization analysis showing various bone marrow states, color-coded by sampling time point or publicly available normal bone marrow data, in longitudinal scRNA-seq profiling of CML with relapse or non-relapse after TKI discontinuation.
FIG. 1D is a graph illustrating the results of a heatmap analysis showing the scaled normalized expression of marker genes for major cell types in longitudinal scRNA-seq profiling of CML with relapse or non-relapse after TKI discontinuation, wherein each column represents a cell and each row represents a marker gene of a major cell type, utilizing the top 100 marker genes for each major cell type.
FIG. 1E is a graph illustrating the proportional distribution of major cell types at various time points in CML patients and normal bone marrow in longitudinal scRNA-seq profiling of CML with relapse or non-relapse after TKI discontinuation, wherein the significance of differential proportions (P-values) between states was determined via a two-sided t-test (box centerline: median; box limits: 25^{th} and 75^{th} percentiles; whiskers: 1.5 times the interquartile range; **p* < 0.05, ***p* < 0.01, ****p* < 0.001).
FIG. 2A is a graph illustrating the results of a UMAP plot analysis showing the expression of marker genes for B cells, erythrocytes, and hematopoietic stem cells (HSCs), which are major cell types, in relation to the characterization of major cell types in CML patients.
FIG. 2B is a graph illustrating the results of a UMAP plot analysis showing the expression of marker genes for megakaryocyte-erythroid progenitors (MEPs), myeloid cells, and natural killer (NK) cells, which are major cell types, in relation to the characterization of major cell types in CML patients.
FIG. 2C is a graph illustrating the results of a UMAP plot analysis showing the expression of marker genes for T cells, megakaryocytes, and mesenchymal stem cells (MSCs), which are major cell types, in relation to the characterization of major cell types in CML patients.
FIG. 2D is a graph illustrating the results of a proportional analysis of major cell types in 86 CML samples, color-coded by each major cell type, in relation to the characterization of major cell types in CML patients.
FIG. 2E is a graph illustrating the results of analyzing the cell counts of major cell types in 86 CML samples, color-coded by each major cell type, in relation to the characterization of major cell types in CML patients, wherein the samples are sorted by state: D_R, S_R, R, D_NR, S_NR, NR, and normal bone marrow.
FIG. 3A is a graph illustrating the results of UMAP visualization analysis of T-cell subsets with cells color-coded by Seurat clusters, in relation to T-cell characterization in CML patients and normal bone marrow.
FIG. 3B is a graph illustrating the results of a heatmap analysis showing the scaled normalized expression of T-cell marker genes, in relation to T-cell characterization in CML patients and normal bone marrow.
FIG. 3C is a graph illustrating the results of a proportional distribution analysis of seven T-cell subsets across various states, in relation to T-cell characterization in CML patients and normal bone marrow.
FIG. 3D is a graph illustrating the results of analyzing the score distribution of a precursor exhaustion signature (left) or a terminal exhaustion signature (right) within each CD8⁺ T-cell cluster, in relation to T-cell characterization in CML patients and normal bone marrow.
FIG. 3E is a graph illustrating the results of a Kaplan-Meier (KM) plot analysis of recurrence-free survival (RFS) based on the proportion of GZMK⁺ IL7R⁻ Tₑₓ cells (left) or T_{reg} cells (right) in an in-house cohort, showing groups with higher proportions of GZMK⁺ IL7R⁻ Tₑₓ cells or T_{reg} cells associated with lower RFS, wherein the high and low groups were divided by the median value of the proportion of each subset, utilizing samples at the time of TKI discontinuation.

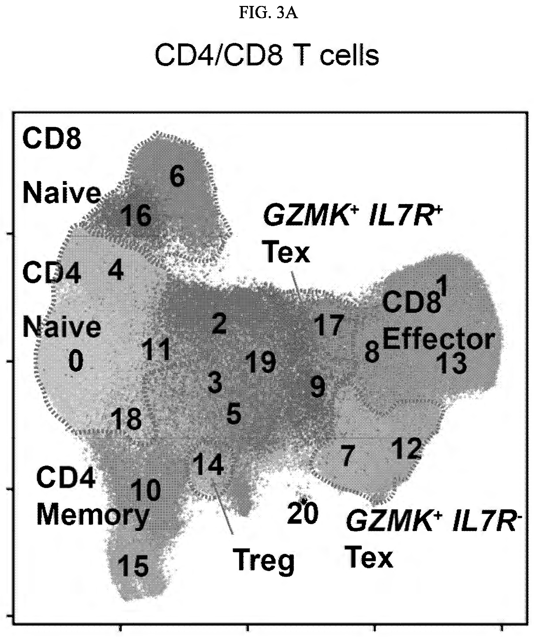

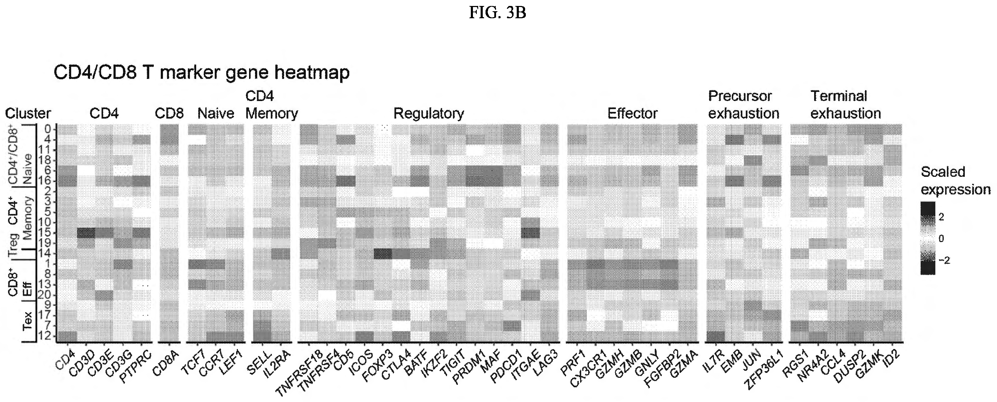

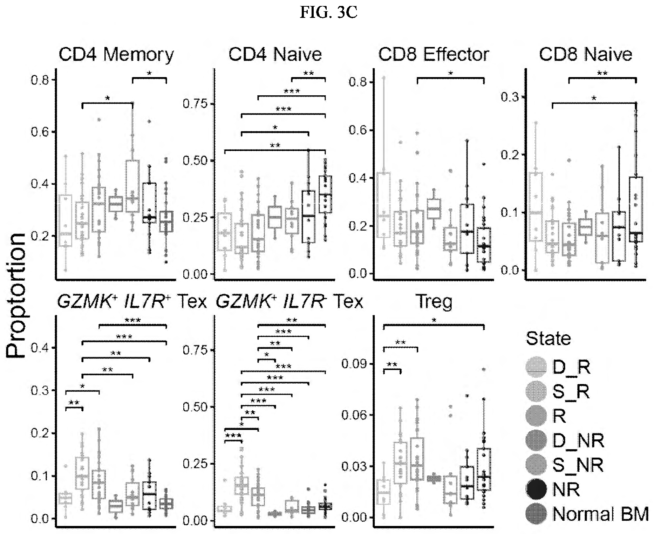

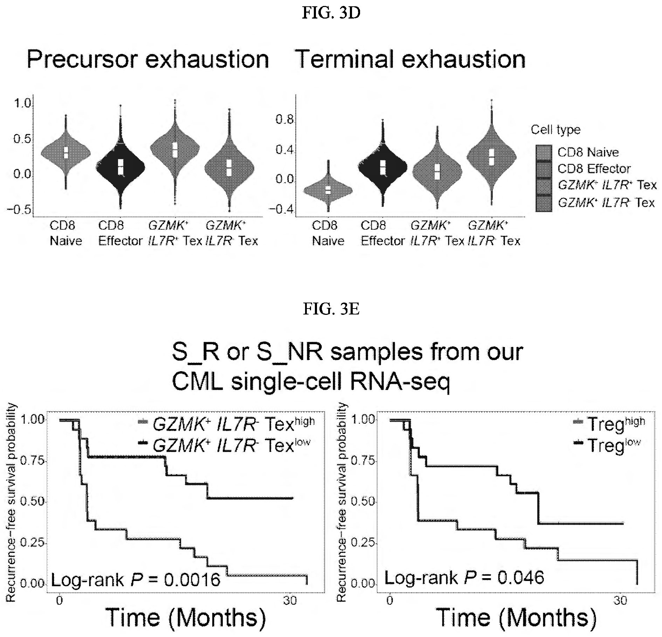

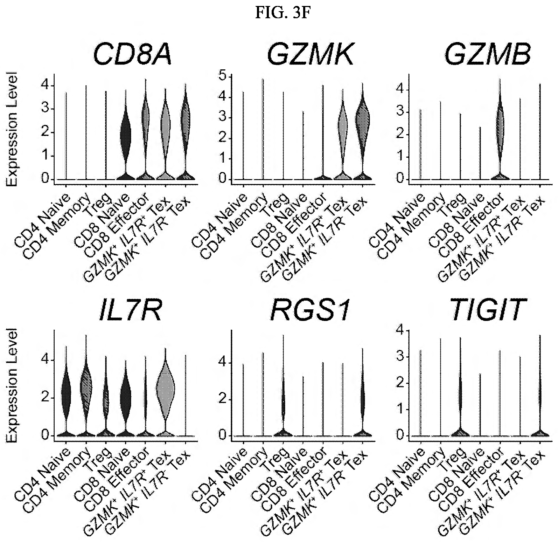

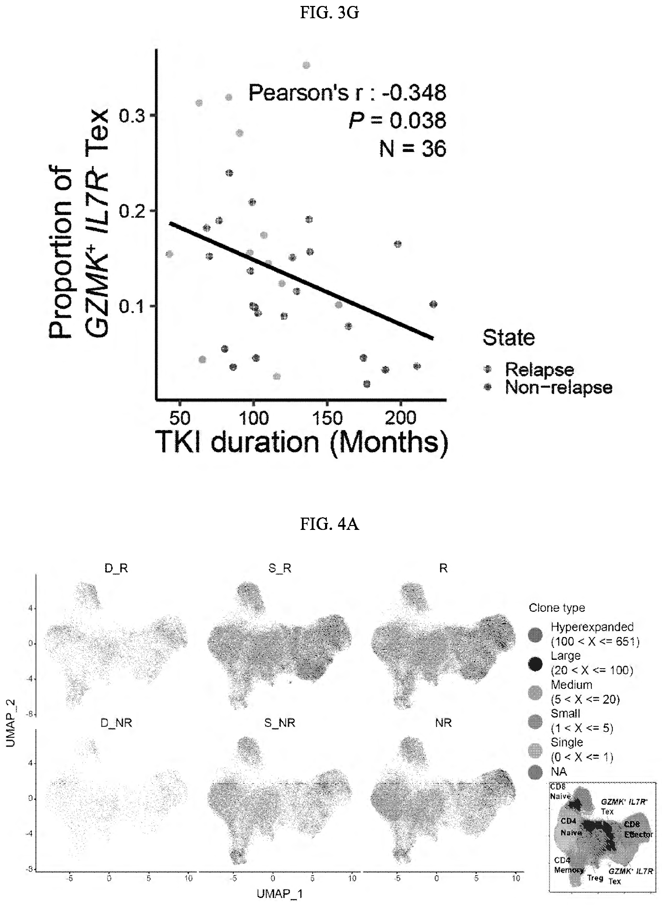

FIG. 3F is a graph illustrating the results of a violin plot analysis showing the expression of genes upregulated or downregulated in GZMK⁺ IL7R⁻ Tₑₓ cells within T-cell subsets, in relation to T-cell characterization in CML patients and normal bone marrow. FIG. 3G is a graph illustrating the results of a scatter plot analysis showing TKI therapy duration versus the proportion of GZMK⁺ IL7R⁻ Tₑₓ cells in samples at the time of TKI discontinuation (n=36), wherein Pearson's correlation coefficient (r) and the associated p-value are reported inside the scatter plot, and the significance of differential proportions (p-values) between states was determined via a two-sided t-test (box centerline: median; box limits: 25^{th} and 75^{th} percentiles; whiskers: 1.5 times the interquartile range; **p <* 0.05, ***p <* 0.01, ****p <* 0.001).
FIG. 4A is a graph illustrating the results of UMAP visualization analysis of T-cell clones at different time points in CML patients, in relation to the clonal expansion of GZMK⁺ IL7R⁻ Tₑₓ cells in S_R and R states of relapsing CML patients (NA: not available).
FIG. 4B is a graph illustrating the results of a quantification analysis of clonotypes within T-cell subsets, in relation to the clonal expansion of GZMK⁺ IL7R⁻ Tₑₓ cells in S_R and R states of relapsing CML patients.
FIG. 4C is a graph illustrating the results of a proportional distribution analysis of expanded clonotypes (defined as 5 or more cells sharing the same TCR) across seven T-cell subsets in different states, in relation to the clonal expansion of GZMK⁺ IL7R⁻ Tₑₓ cells in S_R and R states of relapsing CML patients.
FIG. 4D is a graph illustrating the results of an alluvial plot analysis showing the proportions of clonotypes relative to time points and T-cell subsets for representative CML patients with relapse (01-D59) and non-relapse (DIS-95), in relation to the clonal expansion of GZMK⁺ IL7R⁻ Tₑₓ cells in S_R and R states of relapsing CML patients.
FIG. 4E is a graph illustrating the results of an alluvial plot analysis showing the proportions of clonotypes relative to time points and T-cell subsets for representative CML patients with late relapse (DIS-111 and DIS-119), in relation to the clonal expansion of GZMK⁺ IL7R⁻ Tₑₓ cells in S_R and R states of relapsing CML patients.
FIG. 4F is a graph illustrating the results of a Jaccard index analysis to evaluate similarity between pairwise T-cell subsets, in relation to the clonal expansion of GZMK⁺ IL7R⁻ Tₑₓ cells in S_R and R states of relapsing CML patients.
FIG. 4G is a chord diagram visualizing the interconnections of T-cell subsets across time points, showing unique and shared clones by T-cell subsets, in relation to the clonal expansion of GZMK⁺ IL7R⁻ Tₑₓ cells in S_R and R states of relapsing CML patients, wherein the significance of differential proportions (p-values) between states was determined via a two-sided t-test (box centerline: median; box limits: 25^{th} and 75^{th} percentiles; whiskers: 1.5 times the interquartile range; **p <* 0.05, ***p <* 0.01, ****p <* 0.001).
FIG. 5A is a graph illustrating the results of UMAP visualization analysis of myeloid, erythroid, and stem cell subpopulations, color-coded by Seurat clusters, in relation to the characterization of myeloid, erythroid, and stem cells in CML patients and normal bone marrow.
FIG. 5B is a graph illustrating the results of a heatmap analysis showing the scaled normalized expression of myeloid, erythroid, and stem cell marker genes, in relation to the characterization of myeloid, erythroid, and stem cells in CML patients and normal bone marrow.
FIG. 5C is a graph illustrating the results of a proportional distribution analysis of 11 myeloid, erythroid, and stem cell subpopulations across various states, in relation to the characterization of myeloid, erythroid, and stem cells in CML patients and normal bone marrow.
FIG. 5D is a graph illustrating the results of a KM plot analysis of RFS based on the granulocyte-monocyte progenitor (GMP) proportion in an in-house cohort, showing groups with higher GMP proportions associated with lower RFS, wherein the high and low groups were divided by the median value of the GMP proportion, utilizing samples at the time of TKI discontinuation.
FIG. 5E is a graph illustrating the results of a violin plot analysis showing the expression of genes associated with blast cells or leukemia stem cells within myeloid and stem cell subpopulations, in relation to the characterization of myeloid, erythroid, and stem cells in CML patients and normal bone marrow.
FIG. 5F is a graph illustrating the results of a proportional distribution analysis of HSC subsets across various states, in relation to the characterization of myeloid, erythroid, and stem cells in CML patients and normal bone marrow.
FIG. 5G is a graph illustrating the results of a developmental trajectory analysis of HSCs and GMPs, wherein each dot corresponds to a single cell and is color-coded by cluster label (left) or pseudotime (bottom right), in relation to the characterization of myeloid, erythroid, and stem cells in CML patients and normal bone marrow.
FIG. 5H is a graph illustrating the results of analyzing the score distributions of HSC, GMP, progenitor, and BCR-ABL1 signatures within each HSC and GMP cluster (top) and the pseudotime trajectory (bottom), in relation to the characterization of myeloid, erythroid, and stem cells in CML patients and normal bone marrow, wherein the significance of differential proportions (p-values) between states was determined via a two-sided t-test (box centerline: median; box limits: 25^{th} and 75^{th} percentiles; whiskers: 1.5 times the interquartile range; **p <* 0.05, ***p <* 0.01, ****p* < 0.001).
FIG. 6A is a graph illustrating the results of UMAP visualization analysis of B-cell and plasma cell subsets, color-coded by Seurat clusters, in relation to the characterization of B cells and plasma cells in CML patients and normal bone marrow.
FIG. 6B is a graph illustrating the results of a heatmap analysis showing the scaled normalized expression of B-cell and plasma cell marker genes, in relation to the characterization of B cells and plasma cells in CML patients and normal bone marrow.
FIG. 6C is a graph illustrating the results of a proportional distribution analysis of six B-cell and plasma cell subsets across various states, in relation to the characterization of B cells and plasma cells in CML patients and normal bone marrow.
FIG. 6D is a graph illustrating the results of a KM plot analysis of RFS based on the proportions of pro-B cells (top left), proliferating B cells (top right), and memory B cells (bottom left) in an in-house cohort, wherein the high and low groups were divided by the median value of the proportion of each subset, utilizing samples at the time of TKI discontinuation.
FIG. 6E is a graph illustrating the results of a violin plot analysis showing the expression of genes associated with tumor relapse or tumor suppressor functions within B-cell and plasma cell subsets, in relation to the characterization of B cells and plasma cells in CML patients and normal bone marrow, wherein the significance of differential proportions (p-values) between states was determined via a two-sided t-test (box centerline: median; box limits: 25^{th} and 75^{th} percentiles; whiskers: 1.5 times the interquartile range; **p <* 0.05, ***p <* 0.01, ****p <* 0.001).
FIG. 7A is a graph illustrating the results of UMAP visualization analysis of NK-cell subsets, with cells color-coded by Seurat clusters, in relation to the characterization of NK cells in CML patients and normal bone marrow.
FIG. 7B is a graph illustrating the results of a heatmap analysis showing the scaled normalized expression of NK-cell marker genes, in relation to the characterization of NK cells in CML patients and normal bone marrow.
FIG. 7C is a graph illustrating the results of a proportional distribution analysis of seven NK-cell subsets across various states, in relation to the characterization of NK cells in CML patients and normal bone marrow.
FIG. 7D is a graph illustrating the results of a KM plot analysis of RFS based on the proportion of active mature NK cells in an in-house cohort, wherein the high and low groups were divided by the median value of the active mature NK cell proportion, utilizing samples at the time of TKI discontinuation.
FIG. 7E is a graph illustrating the results of a violin plot analysis showing the expression of genes highly expressed in active mature NK cells within NK-cell subsets, in relation to the characterization of NK cells in CML patients and normal bone marrow, wherein the significance of differential proportions (p-values) between states was determined via a two-sided t-test (box centerline: median; box limits: 25^{th} and 75^{th} percentiles; whiskers: 1.5 times the interquartile range; **p <* 0.05, ***p <* 0.01, ****p <* 0.001).
FIG. 8A is a graph illustrating intercellular correlations identifying cellular subset abundances and prognostic cell types through machine learning to predict relapse status after TKI discontinuation, specifically showing the analysis of correlations between cellular subsets of CML and normal bone marrow samples based on proportions within each major cell type, wherein correlation coefficients (r) and p-values were computed via Pearson's correlation test (**p* < 0.05, ***p* < 0.01, ****p* < 0.001).
FIG. 8B is a schematic diagram illustrating a brief summary of biological factors associated with the pathogenesis of CML or relapse after TKI discontinuation.

### DETAILED DESCRIPTION OF THE INVENTION

### Definitions

As used herein, the term "leukemia" refers to a type of cancer that affects the blood and bone marrow, characterized by the excessive production of abnormal white blood cells. This process interferes with the production of normal blood cells, weakens the immune system, and can lead to various clinical symptoms. Leukemia is broadly classified into acute leukemia and chronic leukemia, with each type further categorized into multiple sub-types. Although the precise etiology of leukemia remains fully elucidated, it is understood to result from a complex interplay of genetic and environmental factors.

As used herein, the term "Chronic Myeloid Leukemia (CML)" refers to a chronic form of leukemia characterized by the overproduction of abnormal white blood cells in the bone marrow, primarily driven by an abnormal chromosome designated as the Philadelphia chromosome. This abnormal chromosome is formed through a reciprocal exchange of segments between chromosomes 9 and 22, generating an abnormal BCR-ABL fusion gene that induces the aberrant proliferation of white blood cells.

As used herein, the term "GZMK" refers to the gene that encodes Granzyme K, an immune-secreted serine protease. It is produced primarily by cytotoxic T cells and natural killer (NK) cells to eliminate infected or cancerous cells and serves as an important regulator of inflammation.

As used herein, the term "IL7R (interleukin-7 receptor)" refers to a protein found on the surface of certain immune cells that regulates the development, survival, and proliferation of B and T cells. It pairs with the cytokine IL-7 to trigger essential chemical signals for healthy immune function.

As used herein, the term "myeloid bias" refers to a preferential tendency to differentiate into specific blood cell types, particularly towards increased production of myeloid cells such as granulocytes or monocytes derived from the bone marrow. In patients with chronic myeloid leukemia, this term specifically indicates an enhanced differentiation of hematopoietic stem cells into the myeloid lineage following the discontinuation of tyrosine kinase inhibitor (TKI) therapy.

As used herein, the term "machine learning" refers to a subset of artificial intelligence characterized by computational algorithms and statistical models that automatically learn underlying mathematical patterns, structures, or non-linear dependencies from a training dataset without being explicitly programmed. Within the framework of the present invention, machine learning algorithms process heterogeneous baseline patient vectors and multi-parametric single-cell transcriptomic biomarkers to perform automated predictive categorization, specifically distinguishing between successful therapeutic remission states and potential molecular relapse risks.

As used herein, the term "Logistic Regression (LR) algorithm" refers to a parametric statistical modeling and supervised classification framework configured to model the probability of a specific binary or dichotomous outcome. In the individual model training pipeline of the present invention, regularized configurations of the LR algorithm-including Ridge (L2 penalty) or Lasso (L1 penalty) regularizations governed by grid search optimization strength-are deployed as binary classifiers within a drug-specific sub-system to determine the prescriptive optimality of a target tyrosine kinase inhibitor (TKI) based on predefined decision boundaries calibrated through validation moving thresholds.

As used herein, the term "Random Forest (RF) algorithm" refers to a supervised ensemble learning method utilizing bootstrap aggregating (bagging) and randomized feature sub-sampling to construct a consensus forest composed of a plurality of independent, randomized decision trees. Individual constituent decision trees within the RF framework sample randomized subsets of clinical and cell-type parameters to output standalone class predictions. Within the operational architecture of the present invention, the RF algorithm is tuned via Bayesian hyperparameter optimization to yield high balanced accuracy (BA) by mapping high-dimensional variables-such as GZMK (granzyme K)⁺ IL7R(interleukin-7 receptor) Tₑₓ cell proportions, blast-specific gene signatures, and ELTS scores-into robust, low-variance binary predictions.

As used herein, the term "3^{rd} generation TKI" refers to a tyrosine kinase inhibitor used for CML patients who have developed resistance or intolerance to earlier treatments, or those whose cancer carries the aggressive T315I gene mutation. These third-generation TKIs include Ponatinib, Olverembatinib, etc.

As used herein, the term "4^{th} generation TKI" refers to a novel tyrosine kinase inhibitor targeting the ABL Myristoyl Pocket. It offers a fundamentally different mechanism of action and superior tolerability compared to older TKIs. Currently, only Asciminib was approved.

### Detailed Description of the Invention

In an aspect of the present invention, there is provided a computer-implemented system for predicting molecular relapse in a patient having chronic myeloid leukemia (CML) undergoing a tyrosine kinase inhibitor (TKI) discontinuation protocol, the system comprising:
a biomarker data input interface configured to receive an immunological data vector generated from a biological sample of a CML patient at a TKI discontinuation milestone or a subsequent relapse monitoring milestone, wherein said data vector comprises a measured proportion of a first exhausted T-cell (Tₑₓ) subpopulation characterized as GZMK⁺ IL7R⁻ Tₑₓ cells and a measured proportion of a second Tₑₓ subpopulation characterized as GZMK⁺ IL7R⁺ Tₑₓ cells, each relative to total CD8⁺ T cells isolated from the biological sample;
a machine learning pipeline processor communicatively coupled to the interface and containing a trained machine learning classification network built on a machine learning algorithm, wherein the pipeline processor is configured to execute automated processing of the data vector by evaluating a numerical equilibrium between the proportion of the GZMK⁺ IL7R⁻ Tₑₓ cells and the proportion of the GZMK⁺ IL7R⁺ Tₑₓ cells to calculate a long-term molecular relapse probability score and classify the patient into either a high molecular relapse risk state or a stable treatment-free remission (TFR) state; and
a therapeutic intervention interface configured to generate and electronically display an interactive clinical dashboard partitioning the patient management profile into a target clinical roadmap according to the classification outputted by the pipeline processor,
wherein the dashboard outputs a specific digital instruction that directs a clinical practitioner to maintain the TKI discontinuation protocol without drug administration when the patient is classified into the stable TFR state, or to execute a predictive frontline TKI resumption regimen when the patient is classified into the high molecular relapse risk state.

In the computer-implemented system, the TKI may be Imatinib, Dasatinib, Nilotinib or Radotinib.

In the computer-implemented system, the machine learning algorithm may be a binary random forest (RF) algorithm or logistic regression (LR) algorithm.

In the computer-implemented system, the proportion of a first exhausted Tₑₓ subpopulation and the proportion of a second Tₑₓ subpopulation may be measured by fluorescence activated cell sorter (FACS), single cell RNA sequencing, and/or single cell TCR sequencing.

In the computer-implemented system, the biological sample may be bone marrow sample or peripheral blood sample and the bone marrow sample may be longitudinal bone marrow sample.

### Model Training, Hyperparameter Selection, and Pipeline Processing

The machine learning pipeline processor of the present invention incorporates a trained machine learning classification network to process the immunological data vector and outputs a clinical classification.

In a preferred embodiment, the classification network is built upon either a Binary Random Forest (RF) algorithm or a Logistic Regression (LR) algorithm, optimized specifically to evaluate the biological interplay and numerical equilibrium between the GZMK⁺ IL7R⁻Tₑₓ and GZMK⁺ IL7R⁺ Tₑₓ cell subpopulations.

### 1. Data Preprocessing and Vector Construction

Prior to model training and inference, raw single-cell data (such as single-cell RNA sequencing (scRNA-seq), single-cell TCR sequencing (scTCR-seq), or fluorescence-activated cell sorting (FACS) data) obtained from a patient's biological sample (e.g., longitudinal bone marrow or peripheral blood) is processed to isolate the CD8⁺ T-cell population. Within this population, the absolute or relative frequencies of the two specific exhausted T-cell (Tₑₓ) subsets-namely GZMK⁺ IL7R⁻ Tₑₓ cells and GZMK⁺ IL7R⁺ Tₑₓ cells-are quantified. These quantified proportions are formatted into a multi-dimensional array, defining the immunological data vector *x =* [*p*₁, *p*₂], where *p*₁ represents the proportion of GZMK⁺ IL7R⁻ Tₑₓ cells and *p*₂ represents the proportion of GZMK⁺ IL7R⁺ Tₑₓ cells relative to the total CD8⁺ T cells.

### 2. Model Training Protocol

The classification network is trained on a retrospective training dataset comprising chronological immunological data vectors obtained from CML patients whose long-term clinical outcomes (either sustaining stable TFR or experiencing molecular relapse) are fully documented.

For the Logistic Regression (LR) model: The network calculates a probability score using a logistic function based on a linear combination of the input vector elements. The decision boundary is established by learning optimal weight coefficients (*w*₁, *w*₂) and a bias (*b*), capturing the log-odds of molecular relapse as a function of the numerical equilibrium between *p*₁ and*p*₂.

For the Random Forest (RF) model: The network constructs an ensemble of independent decision trees during training. Each tree splits the data nodes based on the threshold values of the subpopulation proportions (*p*₁, *p*₂) to maximize information gain (e.g., minimizing Gini impurity or entropy). The final classification output is determined by majority voting across all constituent trees.

To prevent overfitting given the high sensitivity of immunological markers, the training phase employs a stratified *k*-fold cross-validation scheme (e.g., 5-fold or 10-fold cross-validation), ensuring that each fold maintains the same percentage of relapse versus stable TFR cases as the complete dataset.

### 3. Hyperparameter Optimization

To maximize the predictive sensitivity and specificity of the pipeline processor, hyperparameter tuning is executed via an automated grid search or Bayesian optimization framework.

For the Random Forest model, optimized hyperparameters include: the total number of decision trees in the forest (ranging from 100 to 1,000), the maximum depth of each tree to prevent overfitting, the minimum number of samples required to split an internal node, and the maximum number of features considered at each split. For the Logistic Regression model, optimized hyperparameters include: the regularization penalty type (*L*₁ LASSO, *L*₂ Ridge, or Elastic Net), and the regularization strength inverse parameter (*C*-value), which prevents the model from assigning excessive weights to any single noise artifact within the immunological vector.

The final model configuration is selected based on maximizing the Area Under the Curve (AUC), which comprehensively evaluates both sensitivity (True Positive Rate) and specificity (True Negative Rate). Specifically, the hyperparameter tuning aims to maximize the Area Under the Receiver Operating Characteristic curve (AUROC) derived from said sensitivity and specificity, ensuring that the pipeline processor achieves the highest predictive accuracy with minimal false positives and false negatives, even in the presence of baseline patient heterogeneity. Additionally, in target embodiments where patient cohorts exhibit data imbalance, the Area Under the Precision-Recall curve (AUPRC) may further be considered as a supplementary performance metric.

### 4. Interface with the Clinical Dashboard

Once the pipeline processor computes the final long-term molecular relapse probability score via the optimized network, the score is mapped onto a binary classification threshold. If the probability score exceeds a predetermined critical threshold (*T*_crit), the patient is flagged into the "high molecular relapse risk state." If it falls below *T_*crit, the patient is classified into the "stable TFR state." This digital classification is then streamed directly to the therapeutic intervention interface to trigger the respective target clinical roadmap on the interactive dashboard.

In another aspect of the present invention, there is provided a computer-implemented method for predicting a probability of molecular relapse in a chronic myeloid leukemia (CML) patient at a tyrosine kinase inhibitor (TKI) discontinuation milestone, the method comprising:
compiling, via a digital data input interface, an immunological data vector generated from a bone marrow or peripheral blood sample obtained from the CML patient at the TKI discontinuation milestone, wherein the data vector comprises a measured proportion of a first exhausted T-cell (Tₑₓ) subpopulation characterized as GZMK⁺ IL7R⁻ Tₑₓ cells and a measured proportion of a second Tₑₓ subpopulation characterized as GZMK⁺ IL7R⁺ Tₑₓ cells, each quantified relative to total CD8⁺ T cells isolated from the sample; routing the immunological data vector into a trained machine learning classification network executed by a computing processor, the classification network built on a binary Random Forest algorithm;
calculating, via the computing processor, a molecular relapse probability score by executing automated processing of the data vector through the binary Random Forest algorithm to evaluate a non-linear numerical equilibrium between the proportion of the GZMK⁺ IL7R⁻ Tₑₓ cells and the proportion of the GZMK⁺ IL7R⁺ Tₑₓ cells; and
generating, via a data output interface, a digital prognostic risk profile classifying the patient into either a high molecular relapse risk state or a stable treatment-free remission (TFR) state based on the calculated molecular relapse probability score, thereby providing an analytical indicator for assessing treatment-free remission feasibility prior to drug cessation.

In the computer-implemented method, the compiling step may further comprise incorporating a baseline proportion of CD8⁺ naive T cells measured from the identical sample into the immunological data vector,
and wherein the calculating step evaluates the baseline proportion of CD8⁺ naive T cells in combination with the GZMK⁺ IL7R⁻ Tₑₓ cells to yield an augmented balanced accuracy score.

In the computer-implemented method, the generating step may output the digital prognostic risk profile with a mathematically validated balanced accuracy of 87% to 94% based on the numerical equilibrium of the Tₑₓ cell subpopulations.

In the computer-implemented method, the trained machine learning classification network may utilize a bootstrap aggregating ensemble configuration comprising a plurality of independent decision trees, wherein each decision tree evaluates randomized subsets of the cell proportion features and is calibrated via an automated hyperparameter optimization scheme.

In the computer-implemented method, the bone marrow or peripheral blood sample may be a longitudinal bone marrow aspirate sample collected from the CML patient over a sequential time course leading up to the TKI discontinuation milestone.

In another aspect of the present invention, there is provided a method for treating a patient having chronic myeloid leukemia (CML) undergoing a tyrosine kinase inhibitor (TKI) discontinuation protocol, the method comprising:
measuring a specific biomarker combination in a bone marrow or peripheral blood sample obtained from the CML patient at a TKI discontinuation milestone, the biomarker combination comprising a proportion of GZMK⁺ IL7R⁻ Tₑₓ cells and a proportion of GZMK⁺ IL7R⁺ Tₑₓ cells relative to total CD8⁺ T cells;
determining a molecular relapse risk state of the patient based on the measured specific biomarker combination, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
modifying the patient's therapeutic regimen according to the determined molecular relapse risk state, by either:
   (i) resuming or continuing a TKI administration protocol or switching the TKI to a 3rd or 4th generation TKI or providing combination therapy of an adjuvant or an immune check point inhibitor with the TKI when the patient is determined to be in the high molecular relapse risk state, or
   (ii) maintaining the TKI discontinuation protocol when the patient is determined to be in the stable TFR state.

In the method, the TKI may be Imatinib, Dasatinib, Nilotinib or Radotinib.

In the method, the 3^{rd} generation TKI may be Ponatinib or Olverembatinib.

In the method, the 4^{th} generation TKI may be Asciminib.

In the method, the molecular relapse risk state may be determined by a machine learning system and the machine learning system may comprise a logistic regression algorithm or a binary random forest algorithm.

In the method, the bone marrow may be longitudinal bone marrow.

In the method, the proportion of GZMK⁺ IL7R⁻ Tₑₓ cells and a proportion of GZMK⁺ IL7R⁺ Tₑₓ cells relative to total CD8⁺ T cells is measured by fluorescence activated cell sorter (FACS), single cell RNA sequencing, and/or single cell TCR sequencing.

In another aspect of the present invention, there is provided a composition for detecting a specific biomarker combination in a bone marrow or peripheral blood samples obtained from chronic myeloid leukemia (CML) patient undergoing a tyrosine kinase inhibitor (TKI) discontinuation protocol comprising reagents for detecting the specific biomarker combination, wherein the specific biomarker combination comprises a proportion of GZMK⁺ IL7R⁻ exhausted T (Tₑₓ) cells and a proportion of GZMK⁺ IL7R⁺ Tₑₓ cells relative to total CD8⁺ T cells, for use in a method of managing a CML patient to predict molecular relapse after TKI discontinuation, wherein the method comprises:
measuring said proportion of GZMK⁺ IL7R⁻ Tₑₓ cells and said proportion of GZMK⁺ IL7R⁺ Tₑₓ cells in the biological sample obtained from the patient;
determining a molecular relapse risk state of the patient based on the measured specific biomarker combination, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
identifying a therapeutic intervention for the patient, wherein a frontline TKI administration protocol is resumed or the prescription is switched to a 3rd or 4th generation TKI or combination therapy of an adjuvant or an immune check point inhibitor with the TKI when the patient is determined to be in the high molecular relapse risk state, and the TKI discontinuation protocol is maintained without drug administration when the patient is determined to be in the stable TFR state.

In the composition for use, the TKI may be Imatinib, Dasatinib, Nilotinib or Radotinib.

In the composition for use, the 3^{rd} generation TKI may be Ponatinib or Olverembatinib.

In the composition for use, the 4^{th} generation TKI may be Asciminib.

In the composition for use, the molecular relapse risk state of the patient may be determined using a machine learning system, and the machine learning system may comprise a logistic regression algorithm or a random forest algorithm.

In the composition for use, the biological sample may be bone marrow sample or peripheral blood sample and the bone marrow sample may be longitudinal bone marrow sample.

In the composition for use, the reagent may comprise
i) an antibody set comprising antibodies against GZMK, antibodies against IL7R and antibodies against CD8;

2) reagents for single cell RNA sequencing; or
3) reagents for single cell TCR sequencing.

Alternatively, the antibody set may comprises functional fragment of the antibodies or antibody memetics such as VLR, nanobody, V_{H}H, repebody, or monobody.

As used herein, the term "antigen" refers to any substance that induces a state of sensitivity and/or immunoreactivity upon coming into contact with and entering an appropriate cell, and reacts in a demonstrable manner with immune cells and/or antibodies of a subject sensitized as such *in vivo* or *in vitro.*

In the present specification, the term "antigen" may be used interchangeably with the term "immunogen," and preferably denotes a molecule containing one or more epitopes capable of stimulating the host immune system to elicit a secretory, humoral, and/or cellular immune response specific to said antigen.

As used herein, the "antibody," also referred to as an immunoglobulin, refers to a Y-shaped protein produced by plasma cells that is utilized by the immune system to identify or neutralize foreign substances such as bacteria or viruses.

Additionally, a "fragment antigen-binding (Fab)" refers to a fragment generated by cleaving an antibody molecule with papain, which is a dimer of two peptides, V_{H}-CH1 and V_{L}-C_{L}, whereas the other fragment generated by papain is referred to as Fc (fragment crystallizable). As a constituent part of an antibody, the Fab fragment serves to specifically bind to an antigen.

The method for determining the proportion of the T cells present in a blood sample may be selected and used without limitation, provided that it is commonly utilized in the art. Most representatively, the proportion (or frequency) of the T cells in blood can be measured using flow cytometry. A marker (preferably a protein expressed on the cell surface) that is specifically or highly expressed in a target cell population is selected, and a fluorescence-labeled antibody that specifically binds to the marker is reacted with blood cells. The blood cells to which the fluorescence-labeled antibody is bound are sorted according to the labeled fluorescence using a fluorescence-activated cell sorter (FACS), and the proportion is calculated by measuring the number of cells labeled with a specific fluorescence.

Recent advances in single-cell RNA sequencing (scRNA-seq) technology have enabled comprehensive investigations into cellular heterogeneity within the bone marrow microenvironment of hematological malignancies and healthy donors (Warfvinge, R. et al., Blood 129(17): 2384-2394). Through scRNA-seq analysis of CML, inhibitory LGALS9-TIM3 and PVR-TIGIT interactions between NK cells and CML cells have been revealed. Furthermore, another study has noted that inhibitory NK cells accumulate in bone marrow samples of CML patients with primary resistance to imatinib. However, characterization of heterogeneous cellular compositions and their association with TFR in a sequential series of bone marrow samples still remain uninvestigated in CML patients.

At present, the therapeutic objective for Chronic Myeloid Leukemia (CML) patients is to achieve Treatment-Free Remission (TFR), considering that Tyrosine Kinase Inhibitor (TKI) discontinuation is associated with significant medical cost savings (approximately 22 million Euros) and no severe adverse events have been reported (Saussele, S. et al., Lancet. Oncol. 19(6): 747-757. 2018). Recent studies have reported that approximately half of the patients experience molecular relapse following the discontinuation of TKI therapy. The present invention has generated a deep and systematic single-cell atlas from 86 bone marrow samples collected at multiple time points from 39 CML patients who either relapsed or did not relapse after discontinuing TKI therapy. To identify cell types differentially enriched in relapse versus non-relapse, the present inventors collected bone marrow samples from CML patients at the time of CML diagnosis, TKI discontinuation, and relapse or non-relapse, as well as publicly available single-cell transcriptomic data of normal bone marrow. Thereafter, the enrichment of GZMK⁺ IL7R⁻ CD8⁺ T cells, granulocyte-monocyte progenitors (GMPs), pro-B cells, and proliferating B cells was characterized in relapsing CML patients starting from the time point of TKI discontinuation. Furthermore, sample-level correlations among the proportions of these subsets were investigated, elucidating their abundance in S_R and their association with low recurrence-free survival (RFS) in the in-house cohort. Additionally, utilizing a Random Forest algorithm, it was discovered that the proportions of three subsets including GZMK⁺ IL_7R⁻ CD8⁺ T cells highly predict the TFR status following TKI discontinuation. In both relapsing and non-relapsing diseases, B cells and dendritic cells were depleted at the time of the patient's CML diagnosis, whereas megakaryocyte-erythroid progenitors (MEPs) were highly abundant. This is consistent with recently published scRNA-seq}$ analyses of bone marrow samples from healthy donors and CML patients at the time of diagnosis (Krishnan, V. et al., Blood 141(22): 2738-2755. 2023). According to a recent review, B cells and dendritic cells were partially recovered after TKI therapy. Patients who relapsed after TKI discontinuation exhibited a significantly higher proportion of hematopoietic stem cells (HSCs) in total cells than healthy donors at all time points. Myeloid cells were abundant at the time of TKI discontinuation and relapse in relapsing CML patients, whereas erythrocytes and T cells were highly abundant in healthy donors. Furthermore, in the case of CML patients who relapsed after TKI discontinuation, two types of exhausted T (Tₑₓ) cells were present at the highest proportions at the time of TKI discontinuation and at the time of relapse monitoring. Notably, CML patients with a higher proportion of GZMK⁺ IL7R⁻ Tₑₓ cells and a higher level of a terminal exhaustion signature exhibited low recurrence-free survival (RFS). The Tₑₓ cells exhibited a phenotype characterized by high expression of GZMK and low expression of GZMB and IL7R. It is noteworthy that the proportion of these GZMK⁺ IL7R⁻ Tₑₓ cells was negatively correlated with the overall duration of TKI therapy. Prior studies have suggested that the duration of TKI therapy is a key factor predicting successful TFR in chronic-phase CML patients (Nicolini, F. E. et al., Clin. Cancer Res. 25(22): 6606-6613, 2019). It is possible that patients with low levels of GZMK⁺ IL7R⁻ Tₑₓ cells represent a subgroup that can derive the greatest benefit from TKI discontinuation. In accordance with the results, the clonal expansion of GZMK⁺ IL7R⁻ Tₑₓ cells was significantly increased in S_R and R of relapsing CML patients compared to NR of non-relapsing CML patients. It is presumed that these expanded and terminally exhausted CD8⁺ T cells are a primary cause of immune evasion and leukemia stem cell (LSC) proliferation in CML, driving disease relapse when TKI therapy is discontinued. In the future, functional validation using in vitro or in vivo models will be required to elucidate the role of T-cell exhaustion in CML relapse following TKI discontinuation. Moreover, CML patients who relapsed after TKI discontinuation exhibited a significantly higher proportion of GMPs at the time of TKI discontinuation or relapse compared to non-relapsing patients or healthy donors. The GMPs associated with relapse after TKI discontinuation showed high expression of blast-specific genes or leukemia-associated antigens such as AZU1, ELANE, MPO, LYZ, and PRTN3 (Huuhtanen, J. et al., Leukemia 38(1): 109-125, 2024). These GMPs may represent leukemic GMPs responsible for rapid expansion in advanced CML. Therefore, targeting leukemic GMPs may be beneficial for CML patients at a high risk of relapse after TKI discontinuation. Additionally, it was found that pro-B cells, proliferating B cells, and transitional B cells were significantly enriched in relapse samples of CML patients who faced relapse after TKI discontinuation compared to non-relapsing patients. This may be due to the impairment of memory B cells through kinase off-target inhibition of B-cell signaling by TKI, or the presence of BCR-ABL1⁺ B cells in relapsing CML patients. More mechanism-oriented studies are required to elucidate the role of these immature B cells in CML relapse after TKI discontinuation. Co-ecosystem analysis using the proportions of different cell subsets in each sample revealed that GMPs, GZMK⁺ IL7R⁻ Tₑₓ cells, and pro-B cells clustered together and were particularly enriched in relapsing patients. Consequently, by utilizing a Random Forest algorithm based on the proportions of three cellular subsets GZMK⁺ IL7R⁻ Tₑₓ cells GZMK⁺ IL7R⁻ CD8⁺ T cells, and CD8⁺ naïve T cells) at the time of TKI discontinuation and at the time of relapse or non-relapse monitoring, the relapse and non-relapse states were predicted with a balanced accuracy of 87-94%. These results suggest that single cell RNA sequence (scRNA-seq) and single-cell TCR sequencing (scTCR-seq) analyses of bone marrow samples from CML patients are promising for predicting the risk of relapse after TKI discontinuation.

In summary, the present invention relates to a novel marker for predicting the probability of molecular relapse in hematological malignancies, and more particularly, to a marker capable of predicting the probability of relapse after discontinuation of a tyrosine kinase inhibitor (TKI) through longitudinal single-cell transcriptomic analysis of Chronic Myeloid Leukemia (CML). CML is a hematological disease characterized by increased proliferation of undifferentiated cells due to the formation of the BCR-ABL fusion protein, which functions as an active kinase.

The objective of treating CML patients is to achieve TFR while reducing costs without causing severe adverse events after TKI discontinuation. However, recent studies have reported that the successful TFR rate is approximately 40-65%, indicating that nearly half of CML patients experience molecular relapse following TKI discontinuation. To further investigate temporal changes in the bone marrow microenvironment of CML patients, the present inventors collected 86 longitudinal bone marrow samples from CML patients who relapsed or did not relapse after TKI discontinuation and generated a systematic single-cell atlas. Thereafter, scRNA-seq and scTCR-seq were performed on bone marrow samples of CML patients at the time of diagnosis, TKI discontinuation, and relapse or non-relapse to identify various cell types associated with relapse or non-relapse. Utilizing a Random Forest algorithm, it was discovered that the proportions of three subsets including GZMK⁺ IL7R⁻ Tₑₓ cells highly predict the TFR status following the discontinuation of TKI therapy. In the case of CML patients who relapsed after TKI discontinuation, two subtypes of Tₑₓ cells were present at the highest proportions at the time points of TKI discontinuation and relapse. The relapse and non-relapse states were predicted with a balanced accuracy of 87-94% using a Random Forest algorithm based on the proportions of the three cellular subsets GZMK⁺ IL7R⁻ Tₑₓ cells, GZMK⁺ IL7R⁻ CD8⁺ T cells, and CD8⁺ naive T cells) at the time of TKI discontinuation and at the time of relapse or non-relapse monitoring.

In conclusion, the novel marker for predicting the probability of molecular relapse in hematological malignancies according to the present invention can identify patients at a high risk of relapse after TKI discontinuation, thereby enabling the establishment of personalized therapeutic strategies. For example, the continued use of a TKI may be recommended or additional therapeutic options may be provided for patients at a high risk of relapse.

In addition, patients at a low risk of relapse are provided with an opportunity to safely discontinue TKI therapy, thereby contributing to improving the patient's quality of life. Furthermore, novel therapeutic modalities targeting leukemic GMPs can be developed. These cells are closely associated with the risk of relapse, and the probability of relapse can be reduced by eliminating or inhibiting them. Therefore, the present invention can assist in the management and treatment of CML patients, ultimately contributing to improving patient prognosis and preventing disease recurrence.

### Examples

Hereinafter, the present invention will be described in further detail with reference to examples. However, the present invention is not limited to the examples disclosed below, but may be implemented in various different forms. The following examples are provided so that this disclosure will be thorough and complete, and will fully convey the scope of the present invention to those skilled in the art.

### Patient recruitment and ethical approval

Samples were obtained from The Catholic University of Korea, Seoul ST. Mary's Hospital and Uijeongbu Eulji Medical Center. Approvals to collect samples from CML patients were granted by the institutional review board at Uijeongbu Eulji Medical Center, Eulji University (IRB number: UEMC 2021-09-007-002). All patients were given full information of the study and provided written informed consent to participate in the study.

### Study design

A total of 39 patients diagnosed with CML and treated with TKI from April 2003 to July 2023 were recruited in the current study. All patients were given full information of the study and provided the written consent after approval of the institutional review board at Uijeongbu Eulji Medical Center, Eulji University (IRB number: UEMC 2021-09-007-002).

### CML sample collection and single-cell library preparation

Bone marrow samples at the time of CML diagnosis, TKI cessation, and relapse or non-relapse were retrieved. Single-cell expression and TCR libraries were prepared using the Single Cell 5' Reagent Kit v2 (10× Genomics, Pleasanton, CA, United States) according to the manufacturer's protocol (Document # CG000331). Libraries underwent paired-end sequencing on the HiSeq X sequencer (Illumina, San Diego, CA, USA); sequencing was conducted by ROKIT GENOMICS Inc. (Seoul, Korea).

### Processing of scRNA-seq data, integration of public normal BM data, and clustering

The Cell Ranger pipeline (version 6.0.1) was applied for preprocessing of sequencing results, including demultiplexing of sequencing results, barcode processing, read alignment, removing empty droplets, and the generation of gene/barcode matrices (Zheng, G.X. et al., Nat. Commun. 8: 14049, 2017). Reads were mapped to the GRCh38 human reference genome (version GRCh38-2020-A), and the matrix for UMI counts for each cell barcode/gene pair was created for each sample. Cell-free mRNA contamination was estimated and removed using SoupX (version 1.4.5) with default parameters (Young, M.D. et al., Gigascience 9(12): giaal51, 2020). For each sample, Scrublet (version 0.2.1) was used to remove potential doublets, with an expected doublet rate of 0.05 (Wolock, S.L. et al., Cell Syst. 8(4): 281-291, 2019). Cells with a doublet score greater than 0.1 were filtered out.

The Seurat package (version 4.3.0) was used for the downstream analysis (Stuart, T. et al., Cell 177(7): 1888-1902, 2019). Low-quality cells were excluded using the following criteria: 1) cells with more than or equal to 10% of the transcripts originating from the mitochondrial genome, estimated as apoptotic cells; and 2) cells with a minimum detected gene fewer than or equal to 500. The gene expression matrix for each cell was then normalized by total expression, multiplying a scale factor of 10,000, and converted to a natural logarithm using the *NormalizeData* function. The *FindVariableFeatures* function was used to detect highly variable genes using the default setting. The cell-cycle scores were measured using the *CellCycleScore* function to regress this signal from the data. Variations caused by the count of detected transcripts, the mitochondrial gene percent, and the cell-cycle heterogeneity were regressed out using the *ScaleData* function.

The same normalization and doublet filtering method was applied when loading a publicly available scRNA-seq dataset of normal bone marrow (normal BM) samples from healthy donors (Oetjen, K.A. et al., JCI Insight 3(23): e126138, 2018). Batch effects were corrected when integrating the dataset with the publicly available scRNA-seq of normal BM, by scvi-tools (version 0.20.1) implemented in R by the reticulate package (version 1.26), to minimize the potential variations from sample processing (Gayoso, A. et al., Nat. Biotechnol. 40(2): 163-166, 2022). Each batch was considered to be a sample of each patient. The batch-corrected expression values stored in the "scvi" assay were used only for dimension reduction and clustering.

For dimension reduction and clustering analysis, the *FindNeighbors* and *FindClusters* functions were used with the reduction parameter of "scvi" and the resolution being 1.0, respectively. Each cell was projected onto a two-dimensional space and visualized using the RunUMAP function with the reduction parameter of "scvi" and default parameters. This step was repeated for the subclustering analysis of major cell types (the number of PCs being 10 and resolution parameters ranging from 0.5 to 1.0 based on the manual review). Cell type annotation was performed with a manual review of canonical marker genes and SingleR estimation (Aran, D. et al., Nat. Immunol. 20(2): 163-172. 2019).

### Differential gene expression

Differentially expressed genes (DEGs) representing each major cell type were identified using the MAST algorithm⁴³ implemented in the *FindAllMarkers* function with the following criteria: 1) DEGs for each cell subset, by comparing each cell subset to all other cell subsets within the same major cell type. 2) DEGs within each of the annotated cell subsets, by comparing each cluster to other clusters of the same cell subset.

### Visualization of marker genes in major cell types

The marker genes of the major cell types were visualized as a heatmap with the *DoHeatmap* function implemented in the Seurat package. The top 100 marker genes for each major cell type were identified, and the scaled expression of each gene in all cells was plotted on a heatmap. The heatmap of marker genes for each major cell type was visualized using ggplot2 (version 3.3.2).

### Pseudotime trajectory analysis

Pseudotime trajectory analysis was conducted for HSC and GMP cells using the Monocle3 package (version 1.3.1) (Qiu, X. et al., Nat. Methods. 14(3):309-315). The raw UMI count matrices and Seurat object were imported using the *new_cell_data_set* function for downstream analysis. The data were normalized and preprocessed using the *preprocess*_*cds* function in the Monocle3 package with the following parameter: num_dim = 100. Dimension reduction and clustering were performed using the functions *reduce_dimension* and *cluster_cells,* respectively. Cells were ordered in pseudotime along a trajectory using the functions *learn_graph* and *order_cells.* HSCs with the highest HSC score were selected as root cells.

### Calculation of functional module scores

To investigate the functional properties of subsets of T cells or clusters of HSCs and GMPs, the *AddModuleScore* function in the Seurat package was used to calculate the gene signature scores identified in previous studies (Warfvinge, R. et al., Blood. 129(17): 2384-2394. 2017; Giustacchini, A. et al., Nat. Med. 23(6): 692-702, 2017; Lambo, S. et al., Cancer Cell 41(12): 2117-2135, 2023; Im, S.J. et al., Nature 537(7620): 417-421, 2016; Bachireddy, P. et al., Cell Rep. 37(6): 109992, 2016). The genes used to calculate the precursor exhaustion score included *TCF7, MS4A4A, TNFSF8, CXCL10, EEF1B2, ID3, IL7R, JUN, LTB, XCL1, SOCS3, TRAF1, EMB, CRTAM, EEF1G, CD9, ITGB1, GPR183, ZFP36L1, SLAMF6,* and *LY6E;* the terminal exhaustion score included *CD3G, FASLG, ID2, LAG3, RGS1, CCL3, CCL3L1, KIAA1671, SH2D2A, DUSP2, PDCD1, CD7, NR4A2, CD160, PTPN22, ABI3, PTGER4, GZMK, GZMA, MBNL1, VMP1, PLAC8, RGS3, EFHD2, GLRX, CXCR6, ARL6IP1, CCL4, ISG15, LAX1, CD8A, SERPINA3,* and *GZMB.* The genes used to calculate the BCR-ABL1 score were provided by Giustacchini et al. (Nat. Med. 23(6): 692-702, 2017) in Tble 1. Only genes upregulated in BCR-ABL1⁺ stem cells from chronic-phase CML patients at diagnosis with a fold change (log₂(fold change) > 1) considered significant were utilized.

**Table 1**

| Gene list for calculating BCR-ABL1 score | | | | | |
|---|---|---|---|---|---|
| BCR-ABL1 score | Gene | Rank | BCR-ABL1 score | Gene | Rank |
| 13.81035598 | GAS2 | 1 | 0.768276086 | POLR2L | 251 |
| 13.35534198 | SELL | 2 | 0.768116344 | LSM7 | 252 |
| 11.39466777 | ARRDC3 | 3 | 0.768055451 | CRYGD | 253 |
| 7.118243546 | NFKBIA | 4 | 0.767355072 | RAB21 | 254 |
| 6.821468954 | AREG | 5 | 0.766666709 | HNRNPD | 255 |
| 5.292583283 | FOS | 6 | 0.766344278 | STMN1 | 256 |
| 4.470413941 | CTNNB1 | 7 | 0.765931505 | APBB2 | 257 |
| 3.957051769 | MTRNR2L10 | 8 | 0.76362872 | HNRNPA1 | 258 |
| 3.948280561 | HNRNPL | 9 | 0.76262784 | RTCA | 259 |
| 3.835379906 | CLEC4GP1 | 10 | 0.762539753 | RPL26 | 260 |
| 3.811321206 | CRHBP | 11 | 0.762379794 | PIEZO2 | 261 |
| 3.79707943 | MTRNR2L3 | 12 | 0.761737677 | C1orf54 | 262 |
| 3.761061025 | MPL | 13 | 0.761482154 | RPL7A | 263 |
| 3.76102124 | GAS5 | 14 | 0.759782211 | PRSS21 | 264 |
| 3.741892426 | ANXA2 | 15 | 0.758000594 | RPL36AL | 265 |
| 3.719480213 | IFITM 1 | 16 | 0.757192807 | IFITM3 | 266 |
| 3.357300904 | LGALS 1 | 17 | 0.756234434 | ZRANB2 | 267 |
| 3.222630693 | MTRNR2L8 | 18 | 0.754192586 | CRK | 268 |
| 3.195818616 | MTRNR2L4 | 19 | 0.753903812 | ANKRD28 | 269 |
| 3.162410559 | C16orf54 | 20 | 0.753392529 | NHP2 | 270 |
| 3.090394076 | CD164 | 21 | 0.747843308 | CYB5A | 271 |
| 2.951572773 | MTRNR2L1 | 22 | 0.747206509 | TXNIP | 272 |
| 2.796692091 | HNRNPH1 | 23 | 0.746611945 | PHYHD1 | 273 |
| 2.779672357 | SOCS2 | 24 | 0.745474757 | MXRA8 | 274 |
| 2.725752494 | FTL | 25 | 0.74491128 | CDK6 | 275 |
| 2.665248209 | CXCR4 | 26 | 0.743149159 | TRA2B | 276 |
| 2.491120205 | CYP51A1 | 27 | 0.742443355 | GLTP | 277 |
| 2.481562552 | EIF5AL1 | 28 | 0.741472444 | PSMA2 | 278 |
| 2.467113001 | MTRNR2L2 | 29 | 0.739444123 | SYK | 279 |
| 2.427626079 | EIF5A | 30 | 0.737260732 | EIF3H | 280 |
| 2.375468985 | FTH1 | 31 | 0.735055493 | PLIN2 | 281 |
| 2.360665367 | ANXA2P2 | 32 | 0.733443424 | VTRNA1-1 | 282 |
| 2.334797242 | GMNN | 33 | 0.733172114 | OCIAD2 | 283 |
| 2.329892136 | FTH1P3 | 34 | 0.733081942 | IFI27L2 | 284 |
| 2.185304948 | CTSG | 35 | 0.732491408 | MAT2B | 285 |
| 2.086307698 | VIM | 36 | 0.732344072 | PRDX2 | 286 |
| 2.046260295 | STON2 | 37 | 0.73107504 | PDCD6 | 287 |
| 1.944915467 | PF4 | 38 | 0.730386708 | HIST2H2AC | 288 |
| 1.933737329 | RSL1D1 | 39 | 0.724072591 | C14orf2 | 289 |
| 1.922800898 | SPINK2 | 40 | 0.720814715 | CFLAR | 290 |
| 1.913577721 | HLA-E | 41 | 0.720314197 | TOB1 | 291 |
| 1.906910976 | KIAA0125 | 42 | 0.719456816 | XBP1 | 292 |
| 1.857804607 | HOPX | 43 | 0.719013745 | RPL35A | 293 |
| 1.854140954 | DUSP1 | 44 | 0.716793778 | DAND5 | 294 |
| 1.852436404 | AAMDC | 45 | 0.716091531 | TIMM8B | 295 |
| 1.841667389 | ERAP2 | 46 | 0.714011807 | MRPL27 | 296 |
| 1.830191278 | RAB31 | 47 | 0.713405191 | NEDD8 | 297 |
| 1.824082699 | MRPL16 | 48 | 0.712496296 | PSMB9 | 298 |
| 1.821653668 | NOG | 49 | 0.710390036 | WSB1 | 299 |
| 1.797363959 | CALR | 50 | 0.708730091 | GGPS1 | 300 |
| 1.789434867 | MTRNR2L6 | 51 | 0.708203025 | PSMB6 | 301 |
| 1.785401954 | RPL18A | 52 | 0.707948217 | FLJ31306 | 302 |
| 1.692037082 | TSC22D3 | 53 | 0.706754493 | YWHAB | 303 |
| 1.668552272 | LINC00987 | 54 | 0.706232932 | ATPIF1 | 304 |
| 1.653479968 | DNTT | 55 | 0.702472171 | TMEM258 | 305 |
| 1.653351992 | RNA5-8S5 | 56 | 0.701248992 | RPS28 | 306 |
| 1.644451128 | GPR128 | 57 | 0.701109243 | COX7C | 307 |
| 1.637245956 | TRA2A | 58 | 0.700207601 | FKBP3 | 308 |
| 1.628772782 | TMSB10 | 59 | 0.699559532 | ARGLU1 | 309 |
| 1.624806226 | CD69 | 60 | 0.698828183 | HCST | 310 |
| 1.59970947 | GOLGA8A | 61 | 0.697531512 | PPT1 | 311 |
| 1.53777745 | PACERR | 62 | 0.697205118 | JMJD6 | 312 |
| 1.534778824 | ANXA1 | 63 | 0.696405432 | EIF6 | 313 |
| 1.5326662 | RNA45S5 | 64 | 0.695237997 | SDHD | 314 |
| 1.52164625 | IFITM2 | 65 | 0.695020116 | ID2 | 315 |
| 1.51520529 | CXCL11 | 66 | 0.694271696 | ARHGDIB | 316 |
| 1.4920091 | RTN4 | 67 | 0.69378826 | HDC | 317 |
| 1.487512737 | RPL37A | 68 | 0.692748151 | NAA20 | 318 |
| 1.486990278 | GOLGA8B | 69 | 0.692376378 | COX5B | 319 |
| 1.482248051 | LOC101927153 | 70 | 0.691838461 | ZNF689 | 320 |
| 1.478409955 | CD74 | 71 | 0.690661413 | PET100 | 321 |
| 1.45723739 | GNAS | 72 | 0.689378259 | LINC01220 | 322 |
| 1.443474102 | PIP4K2A | 73 | 0.689163464 | PTBP3 | 323 |
| 1.433902931 | LRRC75A-AS1 | 74 | 0.688881976 | FUT1 | 324 |
| 1.422410822 | YBX1 | 75 | 0.688563131 | IDS | 325 |
| 1.401338799 | NCF4 | 76 | 0.687737815 | WIPI1 | 326 |
| 1.395657002 | TSC22D1 | 77 | 0.687227585 | VAMP8 | 327 |
| 1.39257255 | CDC42 | 78 | 0.685326616 | LOC284454 | 328 |
| 1.387538649 | RPL10A | 79 | 0.68285727 | SUMO2 | 329 |
| 1.37363136 | EIF2S3 | 80 | 0.682811337 | LOC101927237 | 330 |
| 1.363161161 | IRF1 | 81 | 0.681763409 | SNRPD2 | 331 |
| 1.348695053 | RSPO1 | 82 | 0.681530692 | SNHG3 | 332 |
| 1.335631618 | DAZAP2 | 83 | 0.680840192 | LOC101928834 | 333 |
| 1.333089378 | AVP | 84 | 0.680490404 | RPL24 | 334 |
| 1.326508667 | C6orf25 | 85 | 0.679519629 | MLLT3 | 335 |
| 1.323938354 | RPS15 | 86 | 0.677859843 | SPATC1L | 336 |
| 1.313119975 | SNHG9 | 87 | 0.677157844 | TBCA | 337 |
| 1.309178656 | PRSS3P2 | 88 | 0.675644441 | SLIRP | 338 |
| 1.307657426 | PROM1 | 89 | 0.675156992 | ZFAS1 | 339 |
| 1.303284246 | PRSS1 | 90 | 0.672503204 | LOC100506844 | 340 |
| 1.298314791 | PCDH9 | 91 | 0.670929877 | MPHOSPH6 | 341 |
| 1.293836951 | HLA-DRB1 | 92 | 0.670289664 | DNAJB6 | 342 |
| 1.283280397 | CD53 | 93 | 0.669893583 | COX5A | 343 |
| 1.242252225 | WTAP | 94 | 0.668745641 | KLF10 | 344 |
| 1.238858617 | CKS2 | 95 | 0.668632668 | ANKRD10 | 345 |
| 1.235224695 | ALG11 | 96 | 0.668546355 | APOBEC3A | 346 |
| 1.226672621 | WDR43 | 97 | 0.664207406 | HBB | 347 |
| 1.221084439 | HMGN3 | 98 | 0.663719194 | PSMB10 | 348 |
| 1.216061161 | MAGED2 | 99 | 0.663266171 | HMGB2 | 349 |
| 1.21484516 | UBB | 100 | 0.661440799 | SGOL1 | 350 |
| 1.194133488 | MRPL55 | 101 | 0.657182393 | C2orf68 | 351 |
| 1.190228895 | ZFP36L2 | 102 | 0.655317157 | SEC61G | 352 |
| 1.187724489 | PSIP1 | 103 | 0.651745635 | COQ10B | 353 |
| 1.182710777 | HBE1 | 104 | 0.651733407 | UCHL3 | 354 |
| 1.168070606 | LIPT1 | 105 | 0.649491483 | SSR4 | 355 |
| 1.161404481 | HIST1H2AC | 106 | 0.649092954 | COX7B | 356 |
| 1.148146587 | HBZ | 107 | 0.646779235 | S100A9 | 357 |
| 1.132928239 | PRDX1 | 108 | 0.646665971 | DPH1 | 358 |
| 1.123917915 | ICAM3 | 109 | 0.646258004 | RHOA | 359 |
| 1.11968186 | MYCT1 | 110 | 0.644720517 | C1orf186 | 360 |
| 1.118500996 | HIST2H2AA3+HIS T2H2AA4 | 111 | 0.642702145 | LINC00963 | 361 |
| 1.115601242 | MT1L | 112 | 0.64211877 | CCNI | 362 |
| 1.109540655 | KLF6 | 113 | 0.640825007 | ZSCAN5A | 363 |
| 1.09612431 | LAPTM4B | 114 | 0.640674151 | ABCC6P1 | 364 |
| 1.092852037 | LOC100507217 | 115 | 0.638367702 | RPL41 | 365 |
| 1.087193318 | TMBIM6 | 116 | 0.636670688 | GSTO1 | 366 |
| 1.072402166 | FXYD5 | 117 | 0.636529073 | PBXIP1 | 367 |
| 1.071694692 | OR13D1 | 118 | 0.636210835 | ST13 | 368 |
| 1.071456111 | SAT1 | 119 | 0.634021386 | DBI | 369 |
| 1.068908482 | CTSC | 120 | 0.633407327 | PRDX3 | 370 |
| 1.051030659 | ID3 | 121 | 0.63324242 | FLT3 | 371 |
| 1.047182683 | EGR1 | 122 | 0.63266047 | SNRPA1 | 372 |
| 1.045646314 | TM7SF3 | 123 | 0.628902608 | SEC22B | 373 |
| 1.034329426 | CDC42SE2 | 124 | 0.627876412 | EIF3M | 374 |
| 1.033317068 | RPL9 | 125 | 0.626028603 | SBDS | 375 |
| 1.032932953 | YWHAZ | 126 | 0.624860548 | USMG5 | 376 |
| 1.02681173 | MARCH6 | 127 | 0.621981672 | SAMD8 | 377 |
| 1.024301574 | PAIP2 | 128 | 0.618867094 | GCSAML-AS1 | 378 |
| 1.024275049 | H3F3B | 129 | 0.618802232 | ZNF283 | 379 |
| 1.022202583 | BST2 | 130 | 0.618267213 | HSPB1 | 380 |
| 1.017771651 | DSEL | 131 | 0.617940848 | CTDP1 | 381 |
| 1.013875279 | COX6A1 | 132 | 0.616292402 | NDUFA12 | 382 |
| 1.01383532 | S100A6 | 133 | 0.615756219 | CARD8 | 383 |
| 0.996889775 | PEBP1 | 134 | 0.615567566 | RGS13 | 384 |
| 0.987516729 | INPP5A | 135 | 0.61516887 | HIST1H4C | 385 |
| 0.984626406 | FHL1 | 136 | 0.613251587 | NDUFS7 | 386 |
| 0.974025717 | FCER1A | 137 | 0.613038109 | RABAC1 | 387 |
| 0.971525704 | RPS11 | 138 | 0.611953796 | TOMM22 | 388 |
| 0.970876654 | TAGLN2 | 139 | 0.610192785 | SORL1 | 389 |
| 0.967354153 | HSPAS | 140 | 0.607652409 | AMICA1 | 390 |
| 0.962493446 | HEMGN | 141 | 0.607308419 | LGALSL | 391 |
| 0.962054267 | S100A4 | 142 | 0.60666234 | TIMP1 | 392 |
| 0.961608377 | SRGN | 143 | 0.604268784 | IQGAP2 | 393 |
| 0.958290947 | PPIA | 144 | 0.60163261 | SCHIP 1 | 394 |
| 0.954016783 | SNIP1 | 145 | 0.598528494 | HIST1H2BN | 395 |
| 0.948447648 | SEPP1 | 146 | 0.598393806 | COMMD3 | 396 |
| 0.947843252 | CNBP | 147 | 0.593732393 | ATP5H | 397 |
| 0.947017584 | SRP14 | 148 | 0.593241463 | H1F0 | 398 |
| 0.944350456 | RPL17 | 149 | 0.592901852 | USP15 | 399 |
| 0.943036127 | C1orf228 | 150 | 0.58839898 | ARPC5 | 400 |
| 0.938010575 | HERC2P2 | 151 | 0.58809095 | DDIT3 | 401 |
| 0.933358727 | LINC00152 | 152 | 0.587325044 | SELK | 402 |
| 0.930902186 | LINC00158 | 153 | 0.584466654 | SELP | 403 |
| 0.930523081 | MMRN1 | 154 | 0.582847755 | PQBP1 | 404 |
| 0.928003757 | FGF18 | 155 | 0.582555592 | CCNG1 | 405 |
| 0.927533503 | CLC | 156 | 0.582347241 | CLNS1A | 406 |
| 0.927523485 | RPL11 | 157 | 0.581092982 | INSR | 407 |
| 0.925333693 | IFI6 | 158 | 0.580907654 | NDUFB4 | 408 |
| 0.914800008 | BCL11A | 159 | 0.577162259 | NGDN | 409 |
| 0.912815533 | PPIL3 | 160 | 0.5737338 | DHRS3 | 410 |
| 0.912192926 | RPS12 | 161 | 0.572620923 | EGR3 | 411 |
| 0.911572736 | SOD2 | 162 | 0.568615317 | KIAA1191 | 412 |
| 0.90892214 | RXFP 1 | 163 | 0.568531549 | MRPS18C | 413 |
| 0.906723812 | NDUFB2 | 164 | 0.568364441 | HBA2 | 414 |
| 0.904307143 | RPS25 | 165 | 0.567418085 | DNAJC24 | 415 |
| 0.901304821 | KCNK6 | 166 | 0.564887855 | HBA1 | 416 |
| 0.900023413 | NDUFB9 | 167 | 0.564187289 | CPNE8 | 417 |
| 0.895646716 | PDLIM1 | 168 | 0.559969057 | CCDC53 | 418 |
| 0.890644079 | NR4A2 | 169 | 0.558665071 | MARCKS | 419 |
| 0.890412911 | UTP11L | 170 | 0.557055578 | DANCR | 420 |
| 0.889437605 | VAV3-AS1 | 171 | 0.556152556 | ARMC8 | 421 |
| 0.88854872 | RPL39 | 172 | 0.554990943 | SERBP 1 | 422 |
| 0.886526682 | HLA-DPA1 | 173 | 0.554371655 | HPR | 423 |
| 0.88649193 | HNRNPA2B 1 | 174 | 0.551448313 | TUBD1 | 424 |
| 0.879635593 | HERC2P9 | 175 | 0.551261504 | HDAC10 | 425 |
| 0.874113373 | PF4V1 | 176 | 0.550799092 | NDUFB3 | 426 |
| 0.873277456 | ACTG1 | 177 | 0.550686774 | HBG1 | 427 |
| 0.87271249 | SKIL | 178 | 0.550236993 | DDX21 | 428 |
| 0.872674398 | PSMB1 | 179 | 0.549227857 | EZH2 | 429 |
| 0.871696857 | TMSB4X | 180 | 0.548262154 | COX7A2L | 430 |
| 0.871553208 | CD52 | 181 | 0.546573147 | MXD1 | 431 |
| 0.870441892 | EGFEM1P | 182 | 0.545769708 | HBD | 432 |
| 0.869651001 | XPNPEP3 | 183 | 0.54275971 | TUBB6 | 433 |
| 0.866680663 | NUDT19 | 184 | 0.541057233 | ID1 | 434 |
| 0.865293386 | C21orf128 | 185 | 0.539565229 | METTL9 | 435 |
| 0.862584016 | GMFG | 186 | 0.53839661 | TEX30 | 436 |
| 0.859504695 | TFRC | 187 | 0.537919833 | RAP1GDS1 | 437 |
| 0.858427715 | NDUFS5 | 188 | 0.537014469 | GLIPR1 | 438 |
| 0.855253463 | CYCS | 189 | 0.536309589 | LINC01021 | 439 |
| 0.85422553 | SNHG15 | 190 | 0.530789818 | CD99 | 440 |
| 0.851624406 | UIMC1 | 191 | 0.530195005 | CECR1 | 441 |
| 0.851574352 | ARPC4 | 192 | 0.529283171 | MT2A | 442 |
| 0.849364535 | SHFM1 | 193 | 0.529051512 | MOCOS | 443 |
| 0.84826139 | LAMP2 | 194 | 0.528700239 | HIST1H2AM | 444 |
| 0.847473094 | MYL6 | 195 | 0.525866866 | DDX1 | 445 |
| 0.839030079 | MDK | 196 | 0.524280388 | NET1 | 446 |
| 0.838984197 | LEPR | 197 | 0.524206445 | OR2AK2 | 447 |
| 0.835299994 | ISCU | 198 | 0.52264049 | TFG | 448 |
| 0.834541931 | KIT | 199 | 0.52260343 | RPP40 | 449 |
| 0.833347634 | HSPA8 | 200 | 0.519940941 | BCYRN1 | 450 |
| 0.832932132 | LRRC70 | 201 | 0.519187049 | PON2 | 451 |
| 0.831600641 | ATP5O | 202 | 0.51597119 | ASF1A | 452 |
| 0.829169039 | PRSS3 | 203 | 0.512407582 | LOC100507530 | 453 |
| 0.829064884 | NDUFB8 | 204 | 0.511848325 | IGFBPL1 | 454 |
| 0.82799919 | ANP32E | 205 | 0.511159013 | TIMM10 | 455 |
| 0.827311523 | HIST1H2BG | 206 | 0.510967082 | ATF4 | 456 |
| 0.827223791 | NGFRAP1 | 207 | 0.510371213 | YIPF1 | 457 |
| 0.825924496 | C12orf65 | 208 | 0.509821493 | RANBP9 | 458 |
| 0.82526277 | NUTF2 | 209 | 0.506928476 | CCDC163P | 459 |
| 0.824589344 | MYL12B | 210 | 0.502910605 | STRA13 | 460 |
| 0.823948938 | STK17B | 211 | 0.502036033 | ZNF595 | 461 |
| 0.823012502 | WDR92 | 212 | 0.501426138 | FDFT1 | 462 |
| 0.821673812 | S100A10 | 213 | 0.500806406 | UMODL1 | 463 |
| 0.819533259 | CCND3 | 214 | 0.500165079 | VSTM1 | 464 |
| 0.818971427 | HLA-DRB6 | 215 | 0.499678708 | SEPT 11 | 465 |
| 0.817694491 | CPSF6 | 216 | 0.49815474 | PDE4B | 466 |
| 0.817574351 | ZNF529 | 217 | 0.496157613 | MGC12916 | 467 |
| 0.815255426 | TERF1 | 218 | 0.494600669 | MT1X | 468 |
| 0.813611349 | CCNL1 | 219 | 0.493716077 | CST3 | 469 |
| 0.808618965 | ATP5A1 | 220 | 0.493079288 | SLC2A13 | 470 |
| 0.807516408 | CRABP1 | 221 | 0.493048079 | TBL1XR1 | 471 |
| 0.805619552 | SMIM14 | 222 | 0.49085837 | CCDC42 | 472 |
| 0.804013978 | ITGA2B | 223 | 0.490113902 | FAXDC2 | 473 |
| 0.802822702 | TLK1 | 224 | 0.489580719 | ROPN1L | 474 |
| 0.799099409 | ADAM28 | 225 | 0.485551343 | NPIPA1 | 475 |
| 0.797559115 | CAPZA1 | 226 | 0.482662351 | TRMT 11 | 476 |
| 0.797553236 | FAM47E | 227 | 0.482026555 | PDCD10 | 477 |
| 0.796159863 | ATP6V0E1 | 228 | 0.477843825 | HBM | 478 |
| 0.795929532 | EIF3I | 229 | 0.476083594 | ANXA5 | 479 |
| 0.795634857 | RPS10 | 230 | 0.474962698 | LRP11 | 480 |
| 0.794731918 | SRSF2 | 231 | 0.470167494 | H3F3A | 481 |
| 0.794338939 | TRAPPC2L | 232 | 0.468979361 | ZNF165 | 482 |
| 0.792585157 | RPA2 | 233 | 0.465620697 | PECAM 1 | 483 |
| 0.792049751 | RPARP-AS1 | 234 | 0.463817247 | GNB1 | 484 |
| 0.790036248 | SSBP1 | 235 | 0.463079372 | SCN9A | 485 |
| 0.789261847 | MYL12A | 236 | 0.458774096 | MRPL46 | 486 |
| 0.788831917 | HNRNPU | 237 | 0.456537522 | MUC4 | 487 |
| 0.785554292 | SLC50A1 | 238 | 0.454148784 | CCDC28A | 488 |
| 0.785255514 | RPS2 | 239 | 0.45387209 | S100A11 | 489 |
| 0.785168573 | H2AFZ | 240 | 0.453843072 | IDH3B | 490 |
| 0.784288227 | TMA7 | 241 | 0.453659992 | PTGIR | 491 |
| 0.780174071 | HMGN2 | 242 | 0.448559489 | KGFLP1 | 492 |
| 0.77796608 | PYCARD | 243 | 0.447788104 | LRRC16A | 493 |
| 0.776539776 | ATP5J | 244 | 0.444235827 | SLC9A3R1 | 494 |
| 0.774798447 | RPS14P3 | 245 | 0.443578335 | WDR89 | 495 |
| 0.772642958 | JAK3 | 246 | 0.441684303 | LSM6 | 496 |
| 0.772397781 | HERC2P7 | 247 | 0.440843198 | C12orf71 | 497 |
| 0.771134252 | FUS | 248 | 0.43974446 | VCP | 498 |
| 0.770529944 | PDIA4 | 249 | 0.439287346 | ICT1 | 499 |
| 0.76892666 | PSMB4 | 250 | 0.438604625 | TMED6 | 500 |

### scTCR-seq data analysis

Clonotypes were visualized and analyzed, and scTCR-seq data were integrated with the Seurat object using the scRepertoire package (version 1.7.0) (Borcherding, N. et al., F1000Res. 9: 47, 2020). First, the list of TCR contigs was combined into clones using the *combineTCR* function. Next, the maximum clone size was calculated using the combination of the nucleotide and gene sequence (CTstrict). After that, the clonal information was added to the Seurat object of T cells using the *combineExpression* function. The distribution of clone types across multiple time points was visualized using the *DimPlot* function in the Seurat package. The *alluvialClonotypes* function was utilized to visualize the proportional contribution of clone types across different states and subsets. Finally, the Jaccard index was calculated using the *clonalOverlap* function to measure the similarity between each of the T-cell subsets.

### Recurrence-free survival analysis

The in-house scRNA-seq and clinical data were obtained to perform the survival analysis. The Kaplan-Meier method and log-rank test were used to analyze recurrence-free survival using the proportions of each subset. In the dataset, samples at the time of TKI cessation were used. The data were divided into two groups using the median of the proportion of each subset. Kaplan-Meier analysis and log-rank tests were performed using the survminer (version 0.4.7) and survival (version 3.2.3) R packages.

### Model training and hyperparameter selection

The present inventors utilized threshold moving on the validation set in individual model training to find the optimal threshold for the probability output of binary classification in LR and RF. Rather than using 0.5 as the default decision threshold to make binary classification, threshold moving finds the appropriate threshold for the particular validation set (Shapley, L. S. et al., Contributions to the Theory of Games, 2: 307-317, 1953). Furthermore, we opted for a Bayesian hyperparameter optimization scheme for RF to tune tree-related hyperparameters using the Optuna package (Branford, S. et al., Haematologica 105(2): 2730, 2020). A grid search scheme was used for LR to find the optimal hyperparameters of LR penalties and the regularization strength parameter. The details of the hyperparameter search scheme are presented in Table 2.

**Table 2**

| Hyperparameters search space | | |
|---|---|---|
| **Model** | **Hyperparameters** | **Method** |
| Logistic Regression | penalty: None, L1, L2 | Grid search |
| | C: 0.1, 0.2, 0.5, 1, 2, 5, 10 | |
| Random Forest | n_estimator: (100, 700) | Bayesian hyperparameter optimization |
| | max_depth: (2,50) | |
| | max_features: ('sqrt', 'log2') | |

### Statistical analysis

All statistical tests were performed in R (version 3.6.3). Pair-wise t-tests were performed to analyze the proportional differences between states within each major cell type/subset. Pearson correlation was used to calculate the correlation between the proportions of each pair of cell subsets. A log-rank test was performed for all RFS analyses to identify significant differences between the high proportion group (top 50%) and the low proportion group (bottom 50%). No mathematical corrections were made for multiple comparisons. Significance is displayed as *, *P* < 0.05; **, *P <* 0.01; and ***, *P* < 0.001.

### Example 1: Longitudinal scRNA-seq of CML Patients with Relapse or Non-Relapse After TKI Discontinuation

A total of 86 bone marrow biopsy samples were collected at various time points from 39 CML patients who either maintained TFR or relapsed after TKI discontinuation (Table 3). Based on the time point of biopsy, the biopsy samples were classified into: D_R (collected at the time of CML diagnosis from relapsing patients), S_R (collected at the time of TKI discontinuation from relapsing patients), R (collected at the time of relapse monitoring from relapsing patients), D_NR (collected at the time of CML diagnosis from non-relapsing patients), S_NR (collected at the time of TKI discontinuation from non-relapsing patients), and NR (collected at the time of relapse monitoring from non-relapsing patients).

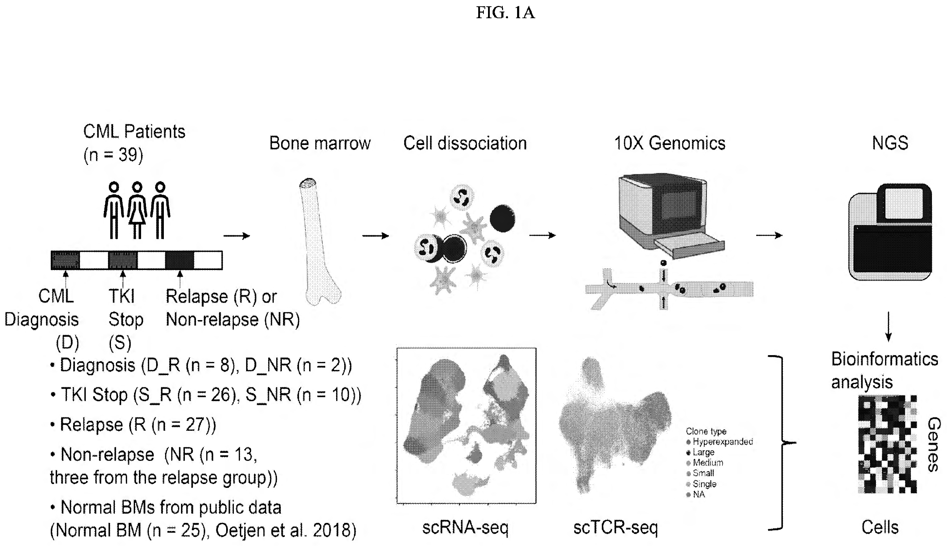

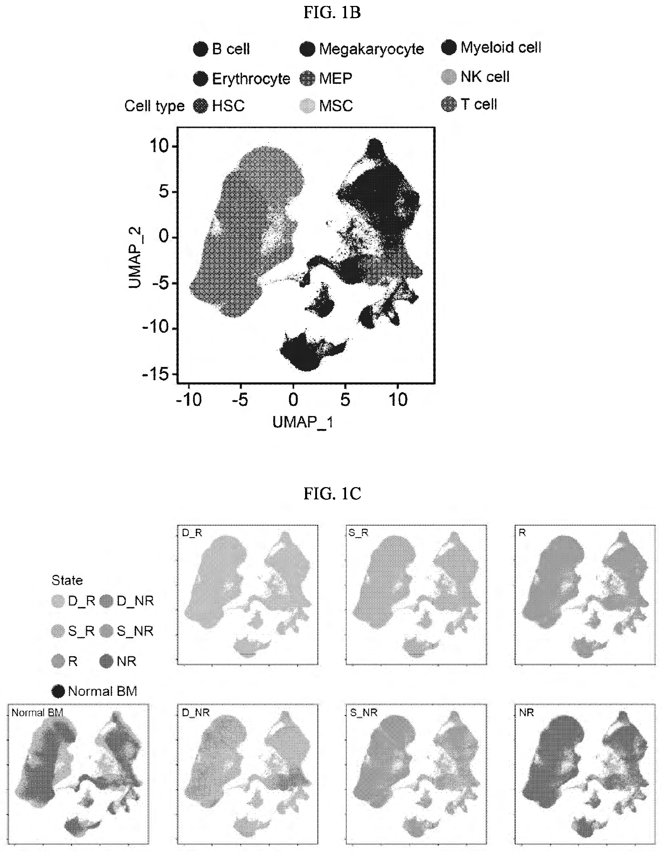

First, bone marrow cells were isolated, and paired scRNA-seq and scTCR-seq were performed utilizing a droplet-based platform (10x Chromium) (FIG. 1A). Overall, an average of 360 million reads were sequenced for each sample, with an average of 1,425 median genes and 3,636 median Unique Molecular Identifiers (UMIs) per cell (Table 1). Additionally, publicly available scRNA-seq data from the normal bone marrow of healthy donors (Normal BM, n = 25) were utilized to compare with the data of the CML bone marrow samples generated in the present invention. After removing low-quality cells and ambient RNA contamination, 509,876 high-quality cells were retained and visualized using UMAP along with 92,384 cells from the normal bone marrow (FIGs. 1B and 1C).

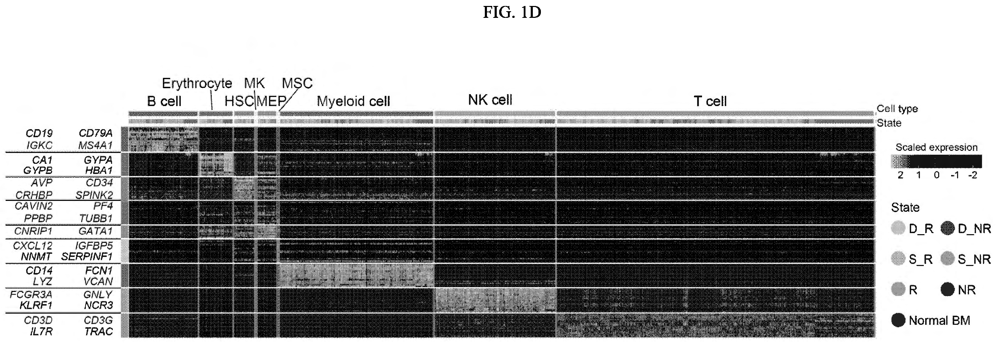

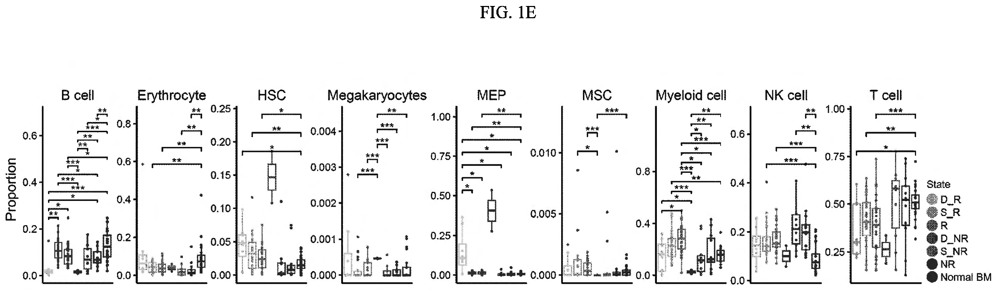

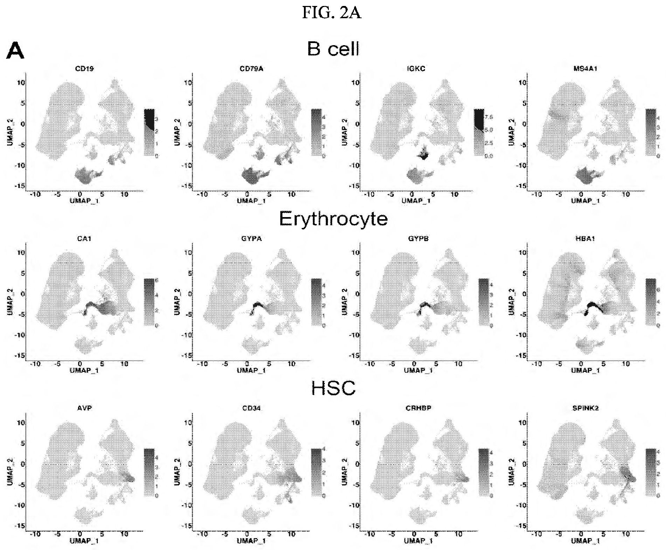

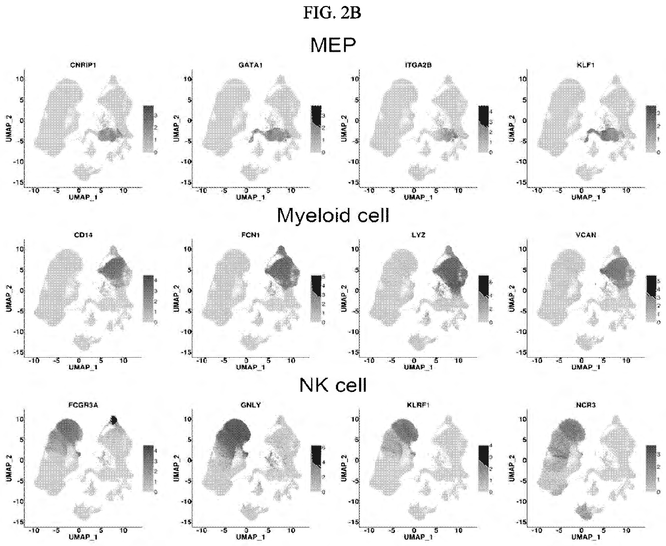

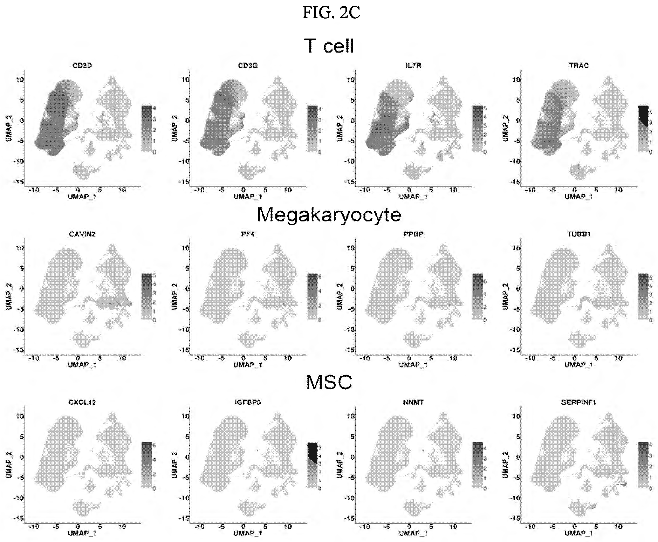

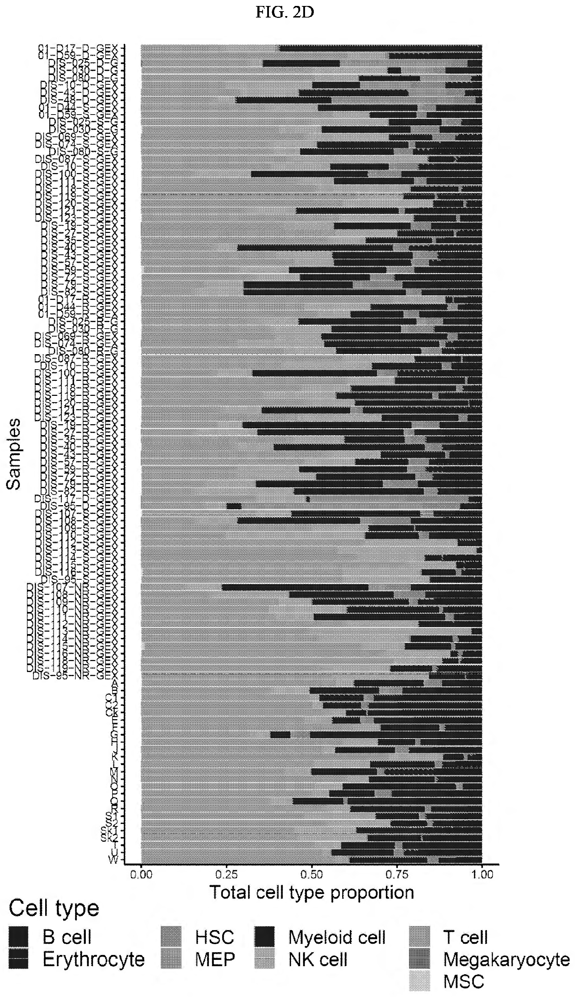

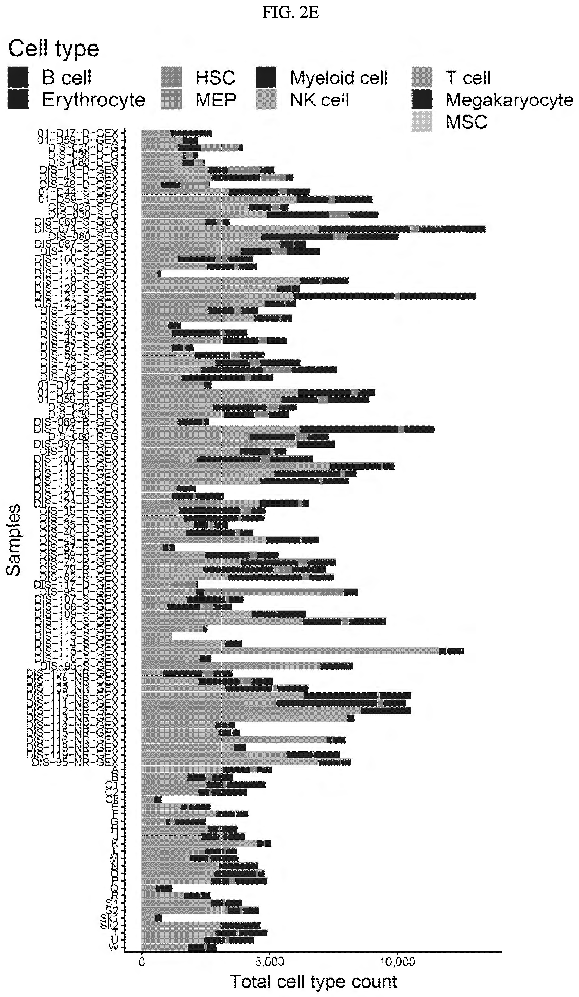

Unsupervised clustering analysis revealed the following 9 major cell types: B cells, erythrocytes, hematopoietic stem cells (HSCs), megakaryocytes (MK), megakaryocyte-erythroid progenitors (MEPs), mesenchymal stem cells (MSCs), myeloid cells, NK cells, and T cells. Major cell types were assigned based on the cell-type-specific expression of established marker genes and SingleR annotations (FIG. 1D, FIGs. 2A to 2C). Compared with other states, depletion of B cells and increased proportions of HSCs and MEPs were observed at the time of CML diagnosis (D_R, D_NR) (FIG. 1E, FIGS. 2D and 2E). Furthermore, it was found that the erythrocyte proportion and NK cell enrichment were significantly reduced at the time of TKI discontinuation (S_R, S_NR) and at the time of relapse monitoring (R, NR) compared to normal bone marrow.

In addition, the proportion of myeloid cells was significantly higher in the samples derived from relapsing patients (R) at the time of relapse monitoring compared to the samples derived from non-relapsing patients (D_NR, S_NR, NR) and the samples of normal bone marrow, which suggests the presence of a "myeloid bias" in the HSCs of CML patients who relapsed after TKI discontinuation. Conversely, samples from relapsing CML patients (D_R, S_R, R) exhibited a significantly lower proportion of T cells compared to samples of normal bone marrow. To better understand the cellular heterogeneity and transcriptional characteristics associated with the relapse of CML patients after TKI discontinuation, as well as temporal changes according to sampling time points and clinical relevance, subclustering analysis was performed on the major cell types. The single-cell RNA sequencing analysis results of the chronic myeloid leukemia samples obtained in the present invention are summarized in Table 3 below.

**Table 3**

| Result of single cell RNA sequencing | | | | | | |
|---|---|---|---|---|---|---|
| **Sample** | **Estimated Number of Cells** | **Mean Reads per Cell** | **Median Genes per Cell** | **Number of Reads** | **Total Genes Detected** | **Median UMI Counts per Cell** |
| 01-D17-D-GEX | 3,857 | 105,174 | 1,206 | 405,657,458 | 20,899 | 3,452 |
| 01-D17-R-GEX | 4,126 | 96,129 | 1,515 | 396,627,437 | 22,101 | 3,774 |
| 01-D44-R-GEX | 10,674 | 38,117 | 1,820 | 406,865,181 | 24,408 | 5,031 |
| 01-D44-S-GEX | 8,102 | 48,803 | 1,539 | 395,402,605 | 24,223 | 4,374 |
| 01-D59-D-GEX | 3,090 | 119,531 | 1,331 | 369,350,393 | 22,250 | 3,010 |
| 01-D59-R-GEX | 10,309 | 38,049 | 1,632 | 392,250,144 | 24,770 | 4,351 |
| 01-D59-S-GEX | 10,675 | 37,736 | 1,819 | 402,835,331 | 24,802 | 4,701 |
| DIS-025-D-G | 4,418 | 90,149 | 2,062 | 398,278,918 | 24,037 | 6,436 |
| DIS-025-R-G | 6,749 | 62,530 | 1,724 | 422,012,950 | 24,598 | 4,260 |
| DIS-025-S-G | 6,636 | 60,066 | 1,894 | 398,597,942 | 24,083 | 4,852 |
| DIS-030-D-G | 3,255 | 125,404 | 1,336 | 408,191,276 | 23,217 | 2,840 |
| DIS-030-R-G | 7,906 | 50,450 | 1,332 | 398,860,922 | 24,983 | 2,798 |
| DIS-030-S-G | 11,107 | 35,744 | 1,530 | 397,013,465 | 25,360 | 3,782 |
| DIS-069-R-GEX | 4,412 | 45,228 | 676 | 199,545,844 | 20,388 | 1,176 |
| DIS-069-S-GEX | 5,085 | 38,663 | 776 | 196,600,665 | 20,862 | 1,462 |
| DIS-074-R-GEX | 14,761 | 21,597 | 1,193 | 318,789,668 | 24,034 | 2,472 |
| DIS-074-S-GEX | 17,012 | 22,644 | 1,020 | 385,218,256 | 24,306 | 2,074 |
| DIS-080-D-G | 3,233 | 123,934 | 1,776 | 400,679,803 | 22,562 | 4,708 |
| DIS-080-R-G | 8,662 | 46,861 | 1,790 | 405,909,369 | 24,144 | 4,670 |
| DIS-080-S-G | 11,670 | 32,837 | 1,542 | 383,209,314 | 24,565 | 4,209 |
| DIS-087-R-GEX | 10,591 | 33,917 | 1,335 | 359,219,591 | 23,026 | 2,891 |
| DIS-087-S-GEX | 9,030 | 33,473 | 1,015 | 302,262,844 | 22,242 | 2,212 |
| DIS-100-R-GEX | 7,908 | 50,363 | 1,163 | 398,273,978 | 22,589 | 2,682 |
| DIS-100-S-GEX | 5,156 | 74,183 | 1,093 | 382,487,774 | 21,538 | 2,654 |
| DIS-107-NR-GEX | 3,970 | 81,583 | 1,598 | 323,883,357 | 21,580 | 4,694 |
| DIS-107-S-GEX | 4,619 | 100,217 | 1,400 | 462,901,835 | 21,894 | 3,959 |
| DIS-108-NR-GEX | 5,931 | 76,721 | 1,290 | 455,031,345 | 22,711 | 3,314 |
| DIS-108-S-GEX | 4,066 | 118,735 | 1,367 | 482,777,696 | 21,736 | 3,606 |
| DIS-109-NR-GEX | 7,674 | 43,024 | 1,720 | 330,167,786 | 23,289 | 4,814 |
| DIS-109-S-GEX | 7,896 | 55,883 | 1,834 | 441,253,308 | 23,001 | 5,047 |
| DIS-10-D-GEX | 6,330 | 58,226 | 1,608 | 368,573,058 | 23,499 | 4,258 |
| DIS-10-R-GEX | 7,702 | 46,657 | 1,310 | 359,349,562 | 22,944 | 3,276 |
| DIS-10-S-GEX | 7,923 | 49,268 | 1,691 | 390,346,707 | 24,200 | 4,803 |
| DIS-110-NR-GEX | 12,499 | 33,430 | 1,618 | 417,843,431 | 23,926 | 4,428 |
| DIS-110-S-GEX | 11,578 | 33,041 | 1,453 | 382,545,704 | 24,135 | 3,276 |
| DIS-111-NR-GEX | 12,300 | 32,922 | 1,521 | 404,943,975 | 23,646 | 4,320 |
| DIS-111-R-GEX | 12,183 | 31,630 | 1,716 | 385,352,600 | 23,288 | 4,963 |
| DIS-111-S-GEX | 6,067 | 68,773 | 1,585 | 417,246,755 | 22,560 | 4,274 |
| DIS-112-NR-GEX | 15,833 | 18,824 | 824 | 298,041,684 | 23,080 | 1,582 |
| DIS-112-S-GEX | 4,308 | 31,844 | 887 | 137,184,780 | 20,174 | 1,816 |
| DIS-113-NR-GEX | 12,549 | 23,999 | 1,485 | 301,162,218 | 22,559 | 3,274 |
| DIS-113-S-GEX | 2,907 | 57,419 | 652 | 166,918,438 | 18,840 | 1,125 |
| DIS-114-NR-GEX | 7,055 | 42,109 | 957 | 297,081,682 | 22,274 | 1,840 |
| DIS-114-S-GEX | 7,159 | 40,551 | 1,261 | 290,305,619 | 22,459 | 2,860 |
| DIS-115-NR-GEX | 5,833 | 44,276 | 1,246 | 258,262,764 | 22,880 | 2,943 |
| DIS-115-S-GEX | 17,482 | 20,609 | 1,627 | 360,293,979 | 23,795 | 4,188 |
| DIS-116-NR-GEX | 11,085 | 30,571 | 1,602 | 338,876,091 | 23,658 | 4,009 |
| DIS-116-S-GEX | 6,699 | 31,778 | 796 | 212,881,073 | 22,278 | 1,443 |
| DIS-117-D-GEX | 4,642 | 38,806 | 1,271 | 180,137,371 | 22,429 | 2,790 |
| DIS-118-NR-GEX | 5,552 | 50,845 | 889 | 282,290,375 | 20,057 | 2,000 |
| DIS-118-R-GEX | 10,052 | 34,887 | 1,576 | 350,679,243 | 22,891 | 4,195 |
| DIS-118-S-GEX | 2,551 | 35,628 | 432 | 90,886,439 | 17,943 | 795 |
| DIS-119-NR-GEX | 9,726 | 30,452 | 1,190 | 296,178,544 | 22,673 | 2,771 |
| DIS-119-R-GEX | 9,152 | 39,606 | 1,966 | 362,478,056 | 23,949 | 5,677 |
| DIS-119-S-GEX | 10,889 | 32,676 | 1,256 | 355,812,392 | 22,770 | 2,936 |
| DIS-120-R-GEX | 4,034 | 42,558 | 624 | 171,680,892 | 19,962 | 1,054 |
| DIS-120-S-GEX | 9,242 | 40,835 | 1,124 | 377,392,623 | 22,397 | 2,388 |
| DIS-121-R-GEX | 3,607 | 101,030 | 2,480 | 364,413,425 | 23,993 | 6,796 |
| DIS-121-S-GEX | 16,400 | 22,761 | 1,035 | 373,275,846 | 24,010 | 2,228 |
| DIS-123-R-GEX | 11,197 | 37,277 | 988 | 417,395,894 | 23,116 | 1,942 |
| DIS-123-S-GEX | 9,020 | 41,424 | 1,058 | 373,645,336 | 22,912 | 2,094 |
| DIS-19-R-GEX | 5,584 | 68,880 | 1,161 | 384,624,461 | 21,542 | 2,819 |
| DIS-19-S-GEX | 5,625 | 72,260 | 1,314 | 406,460,823 | 22,156 | 3,704 |
| DIS-27-R-GEX | 5,592 | 69,405 | 1,482 | 388,115,244 | 22,258 | 4,266 |
| DIS-27-S-GEX | 7,428 | 54,799 | 960 | 407,049,162 | 20,628 | 2,445 |
| DIS-35-R-GEX | 3,930 | 101,690 | 1,893 | 399,642,777 | 23,030 | 4,748 |
| DIS-35-S-GEX | 2,052 | 191,619 | 1,812 | 393,201,503 | 21,234 | 4,041 |
| DIS-40-R-GEX | 5,193 | 76,049 | 1,031 | 394,921,156 | 21,432 | 2,399 |
| DIS-40-S-GEX | 4,805 | 75,260 | 1,034 | 361,624,194 | 21,041 | 2,288 |
| DIS-43-D-GEX | 6,959 | 56,400 | 1,418 | 392,485,387 | 23,047 | 3,663 |
| DIS-43-R-GEX | 8,914 | 41,911 | 1,715 | 373,590,327 | 23,094 | 4,506 |
| DIS-43-S-GEX | 6,543 | 57,960 | 1,773 | 379,232,927 | 23,714 | 4,733 |
| DIS-48-D-GEX | 3,061 | 131,913 | 1,766 | 403,786,262 | 22,923 | 4,568 |
| DIS-57-R-GEX | 1,484 | 254,374 | 1,962 | 377,491,099 | 21,316 | 4,788 |
| DIS-57-S-GEX | 2,523 | 156,502 | 1,595 | 394,855,030 | 21,833 | 3,943 |
| DIS-59-R-GEX | 6,428 | 59,473 | 1,707 | 382,291,181 | 23,226 | 4,745 |
| DIS-59-S-GEX | 5,954 | 60,949 | 1,388 | 362,888,688 | 23,203 | 3,508 |
| DIS-72-R-GEX | 8,830 | 45,440 | 1,598 | 401,231,718 | 23,947 | 3,977 |
| DIS-72-S-GEX | 7,215 | 51,615 | 1,563 | 372,400,160 | 23,791 | 3,760 |
| DIS-79-R-GEX | 8,338 | 50,893 | 1,265 | 424,344,868 | 22,716 | 3,502 |
| DIS-79-S-GEX | 8,792 | 45,138 | 1,250 | 396,851,079 | 22,983 | 3,354 |
| DIS-82-R-GEX | 8,853 | 46,603 | 1,054 | 412,574,838 | 22,325 | 2,669 |
| DIS-82-S-GEX | 6,050 | 70,081 | 1,393 | 423,989,332 | 21,784 | 3,756 |
| DIS-95-D-GEX | 9,964 | 38,202 | 3,654 | 380,643,516 | 24,102 | 13,872 |
| DIS-95-NR-GEX | 10,168 | 38,279 | 1,910 | 389,225,384 | 23,306 | 5,140 |
| DIS-95-S-GEX | 9,899 | 38,658 | 1,790 | 382,672,633 | 22,858 | 4,875 |

### Example 2: Characterization Analysis of T Cells in CML Patients and Normal Bone Marrow

The characterization of T cells in CML patients and normal bone marrow was analyzed. As a result, based on canonical marker gene expressions, T cells were classified into 21 clusters comprising CD4⁺, CD8⁺, naive, CD4 Memory, regulatory T cells (T_{reg}), CD8 effectors, and exhausted CD8⁺ T cells (Tₑₓ). Since leukemia stem cells are known to persist in CML patients despite imatinib treatment, it was hypothesized that immunosuppressive T-cell subpopulations would be prevalent in CML patients who relapsed after TKI discontinuation. Importantly, GZMK⁺ IL7R⁺ Tₑₓ and GZMK⁺ IL7R⁻ Tₑₓ cells were found to be significantly enriched in the relapse patient groups S_R and R compared to non-relapse patients or normal bone marrow. Conversely, the proportion of CD4 Memory T cells was higher in S_NR (non-relapse patients), and naive T cells were significantly abundant in normal bone marrow.

In addition, cells with the highest terminal exhaustion score were predominantly found in GZMK⁺ IL7R⁻ Tₑₓ, whereas cells with the highest precursor exhaustion score were mainly present in GZMK⁺ IL7R⁻ CD8⁺ Tₑₓ. Notably, a high proportion of GZMK⁺ IL7R⁻ Tₑₓ cells at the time point of TKI discontinuation was associated with a low recurrence-free survival (RFS) rate (Log-rank P = 0.0016). Furthermore, the group with a higher proportion of T_{reg} cells also exhibited a lower RFS (Log-rank P = 0.046). These GZMK⁺ IL7R⁻ Tₑₓ cells, exhibiting a terminally exhausted phenotype, showed high expression of GZMK and low expression of GZMB, which may indicate pre-dysfunctional T cells and the upregulation of known T-cell exhaustion markers RGS1 and TIGIT. The duration of TKI therapy demonstrated a negative correlation with the proportion of GZMK⁺ IL7R⁻ Tₑₓ cells. In summary, GZMK⁺ CD8 T cells possess a terminally exhausted phenotype and were enriched in relapsed CML patients starting from the time point of TKI discontinuation.

### Example 3: Clonal Expansion of GZMK⁺ IL7R⁻ Tₑₓ Cells in S R and R States of Relapsing CML Patients

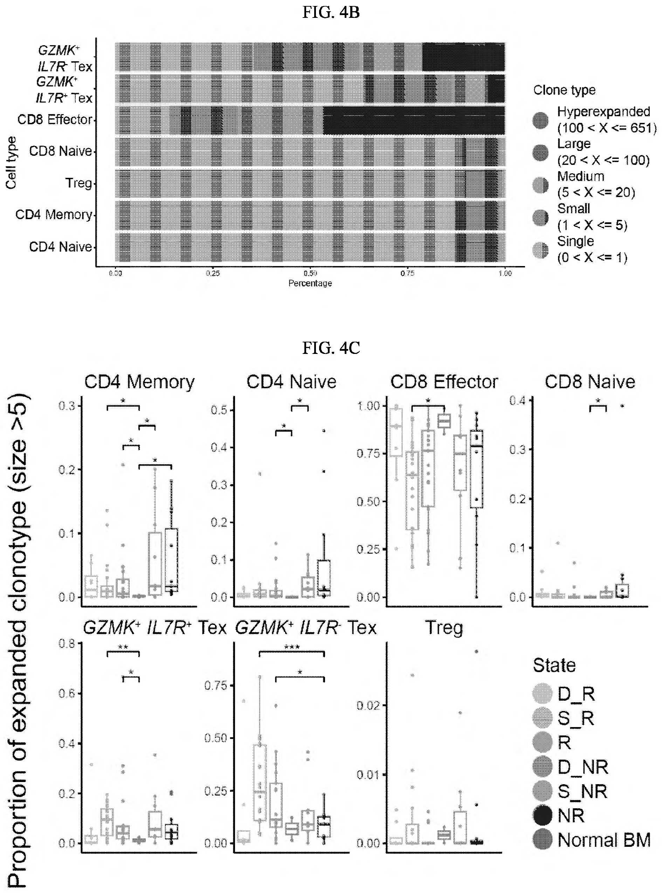

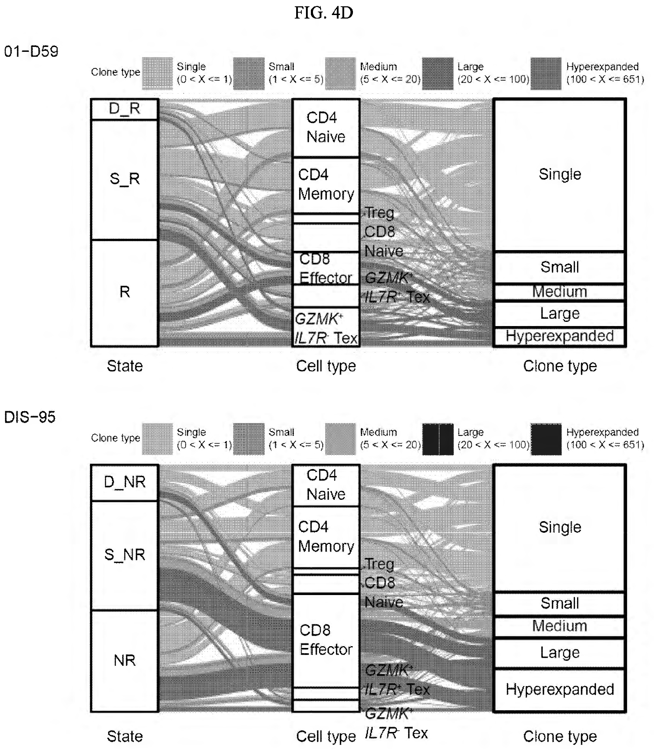

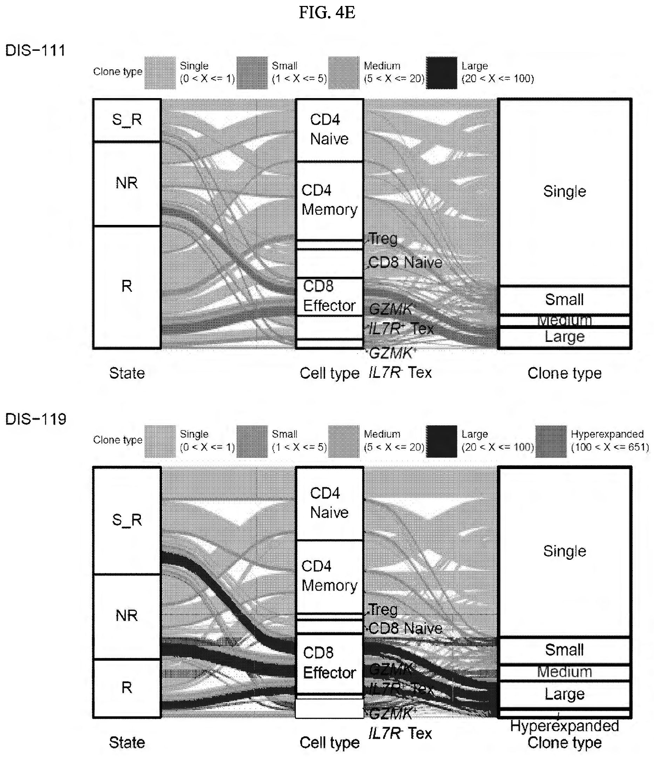

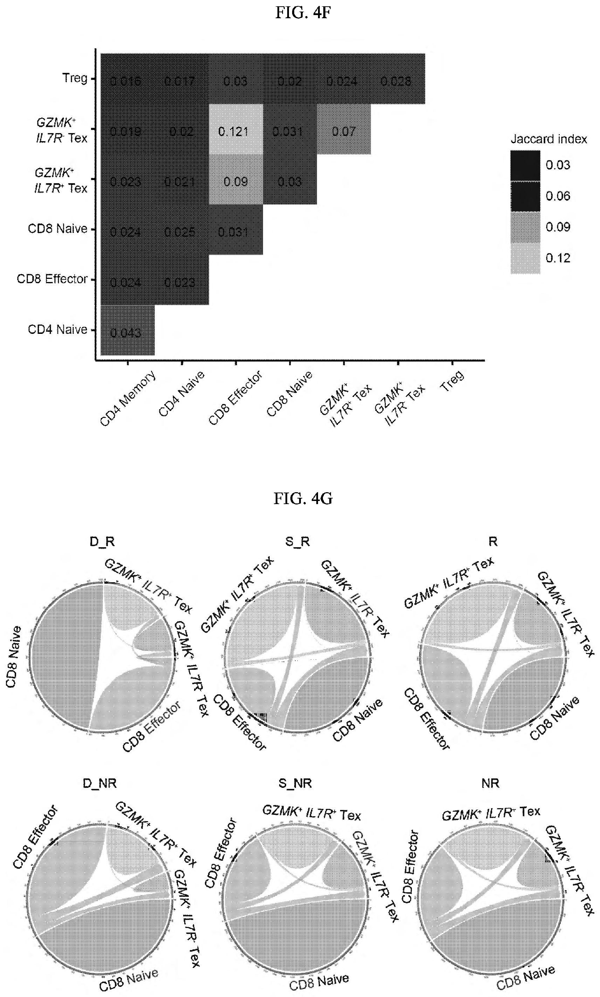

The clonal distribution of T cells was investigated to further identify T-cell-associated factors linked with CML relapse. The clonal distribution across all time points demonstrated that clonally expanded cells (defined as five or more cells sharing the same TCR) were predominantly mapped to CD8⁺ effectors and Tex cells. Conversely, CD4 naive, CD4 memory, T_{reg}, and CD8 naive cells were primarily unexpanded cells or minimally expanded cells detected in five or fewer cells (FIGs. 4A and 4B). The proportional distribution of expanded clonotypes indicated that clonally expanded GZMK⁺ IL7R⁻ Tₑₓ cells were enriched in samples derived from relapsing CML patients (S_R and R) compared to samples derived from non-relapsing patients (NR) (FIG. 4C). For example, the sample of 01-D59, which is a sample derived from a relapsing patient (S_R and R), exhibited a high prevalence of hyperexpanded clones in GZMK⁺ IL7R⁻ Tₑₓ cells (FIG. 4D). In contrast, in the sample of DIS-95, which represents the non-relapsing patient group (S_NR and NR), hyperexpanded clones were highly expressed in CD8⁺ effectors. Furthermore, among patients with late relapse (defined as relapse occurring 6 months or more after TKI discontinuation), there were three patients for whom samples were available at both non-relapse and relapse time points. Although one patient exhibited a low T-cell count in NR (data not shown), clonal expansion of CD8⁺ effectors was also observed in NR and R of the two late-relapse patients. Next, an analysis of TCR repertoire overlap between cell types was performed, and as a result, the TCR repertoire was found to partially overlap between CD8⁺ effectors and Tₑₓ cells, with the highest Jaccard index observed in GZMK⁺ IL7R⁻ Tₑₓ cells (FIG. 4E). By analyzing the TCR repertoire shared among CD8⁺ T cells, an active transition from CD8⁺ effector T cells to Tₑₓ cells across all time points was inferred (FIG. 4F). In summary, clonally expanded GZMK⁺ IL7R⁻ CD8⁺ exhausted T cells are overrepresented at the time of TKI discontinuation and relapse in relapsing CML patients.

### Example 4: Enrichment of GMPs and HSCs Carrying High BCR-ABL1 Scores at the Time of TKI Discontinuation in Relapsing CML Patients

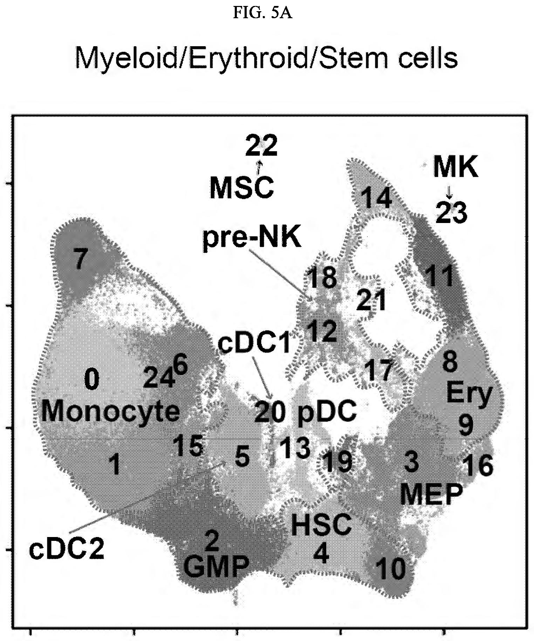

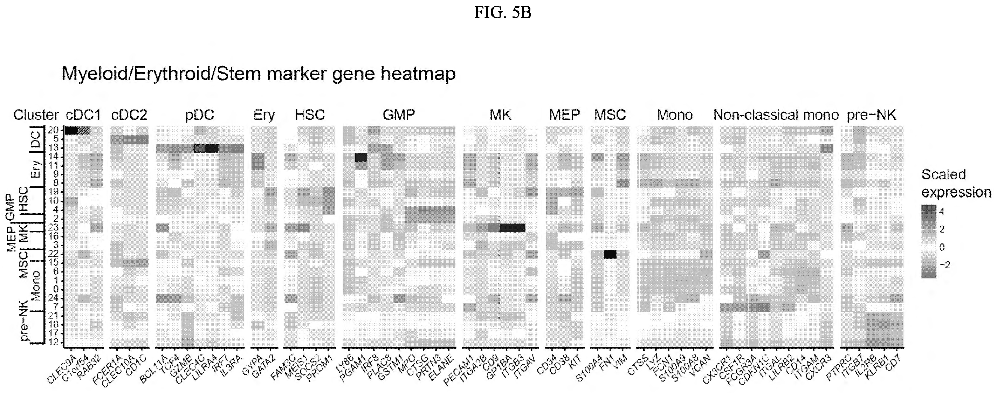

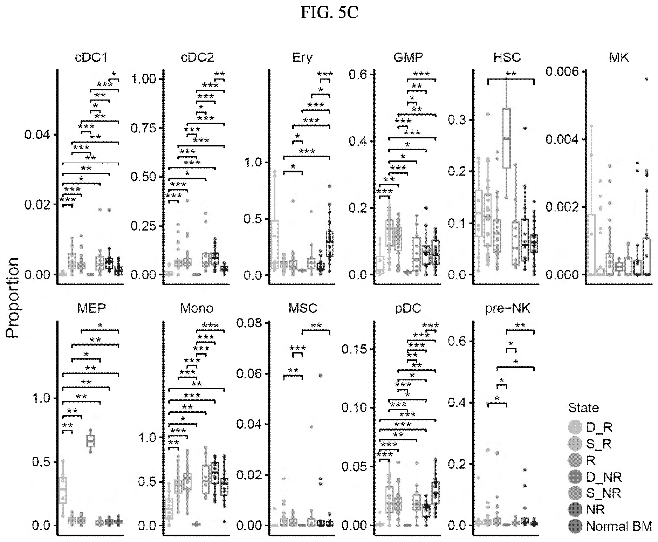

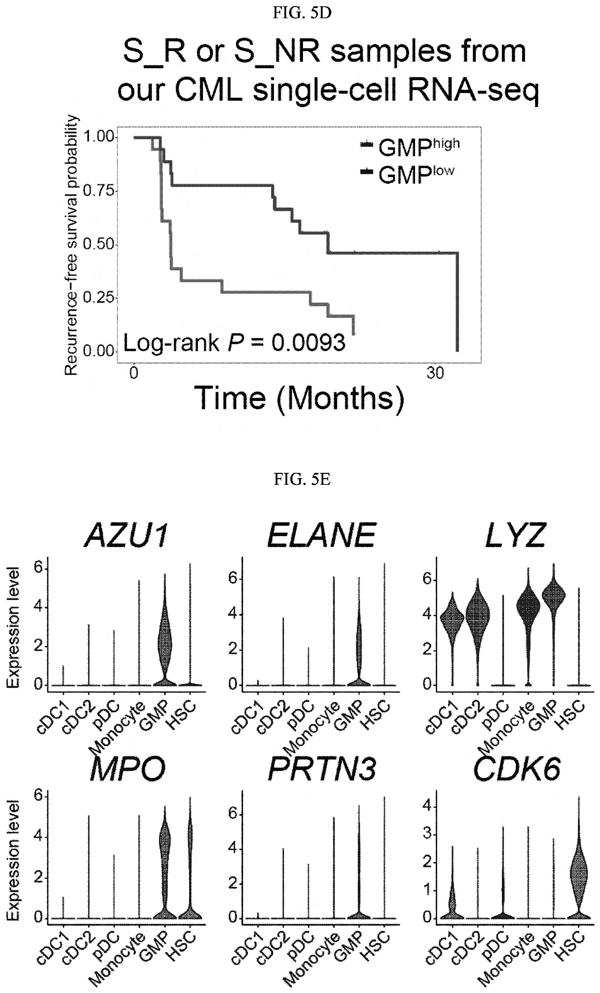

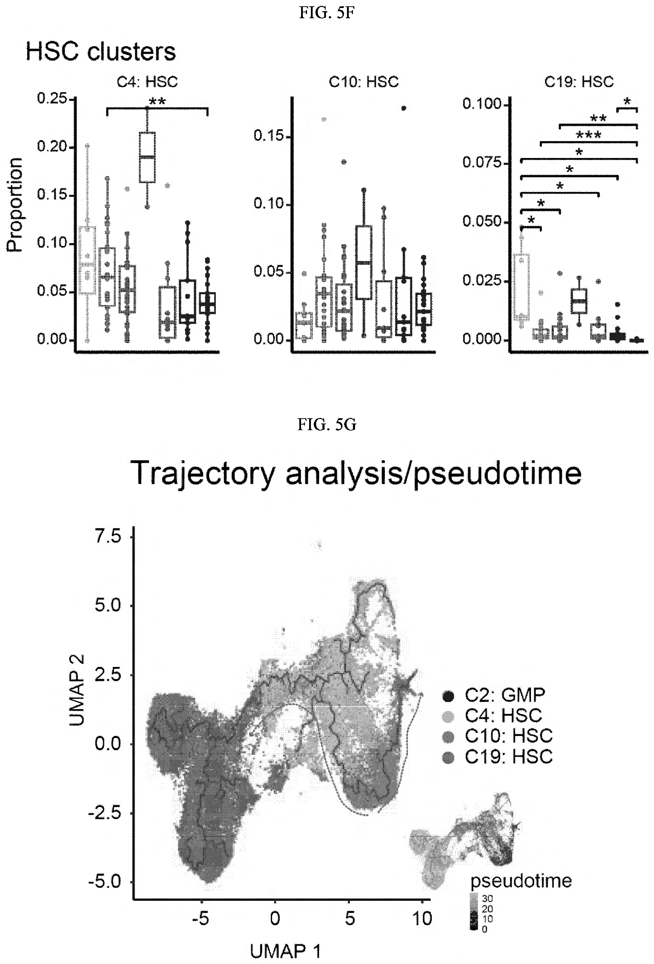

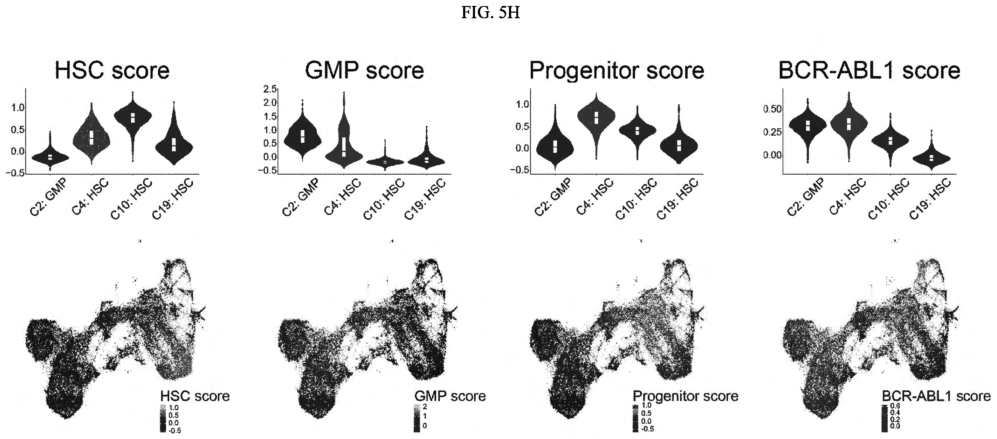

Subclustering analysis was performed on bone marrow, erythroid, and stem cells to further investigate HSC and myeloid lineage cell types associated with relapse or non-relapse following TKI discontinuation. Based on canonical markers and SingleR annotations, various cell types were identified, including dendritic cells (DCs), erythrocytes (Ery), HSCs, granulocyte-monocyte progenitors (GMPs), megakaryocytes (MK), MEPs, MSCs, monocytes (Mono), and NK cell precursors (pre-NK) (FIGs. 5A and 5B). Consistent with prior studies, DCs consisting of type 1 classical DCs (cDC1), type 2 classical DCs (cDC2), and plasmacytoid DCs (pDCs) were depleted at the time of CML diagnosis and recovered by TKI treatment, whereas MEPs were expanded at the time of CML diagnosis (FIG. 5C) (Krishnan, V. et al., Blood 141(22): 2738-2755. 2023). The proportion of erythrocytes was significantly lower at the time of TKI discontinuation, relapse, and non-relapse compared to normal bone marrow, and was enriched in S_R. Individuals with a higher GMP proportion at the time of TKI discontinuation exhibited lower RFS (Log-rank P = 0.0093) (FIG. 5D). GMPs specifically demonstrated upregulation of blast-specific or leukemia-associated genes such as AZU1, ELANE, LYZ, MPO, and PRTN3 (FIG. 5E) (Mumme, H, et al., Nat. Commun. 14(1): 6209, 2023). HSCs overexpressed CDK6, which is known as a key regulator of HSC and leukemia stem cell (LSC) activation (Scheicher, R. et al., Blood 125(1): 90-101. 2015). To investigate the heterogeneity of the stem cell population, HSCs were separated into three clusters. C19: HSCs were abundant at the time of CML diagnosis, reflecting that hematopoietic stem and progenitor cells were expanded during leukemogenesis (FIG. 5F). Notably, C4: HSCs were more abundant at the time of TKI discontinuation in relapsing CML patients compared to normal bone marrow. In addition, trajectory analysis of HSCs and GMPs was performed to elucidate the association between heterogeneous HSCs and GMPs at various time points (FIG. 5G). Two pathways were identified: (I) one from C10: HSCs to C4: HSCs and then to GMPs, and (II) the other from C10: HSCs to C19: HSCs. As a result, C10: HSCs exhibited the highest HSC score among the clusters, whereas C4: HSCs demonstrated the highest progenitor score (FIG. 5H). GMPs and C10: HSCs exhibited the highest BCR-ABL1 scores compared to other clusters, implying BCR-ABL1-induced transformation of leukemic GMPs and LSCs (Minami, Y. et al., Proc. Natl. Acad. Sci. USA. 105(46): 17967-17972, 2008). Taken together, these results indicate that leukemic GMPs and LSCs are present at a high frequency in relapsing CML patients at the time of TKI discontinuation.

### Example 5: Quantitative Patterns of B-Cell Subsets in CML Patients and Healthy Donors

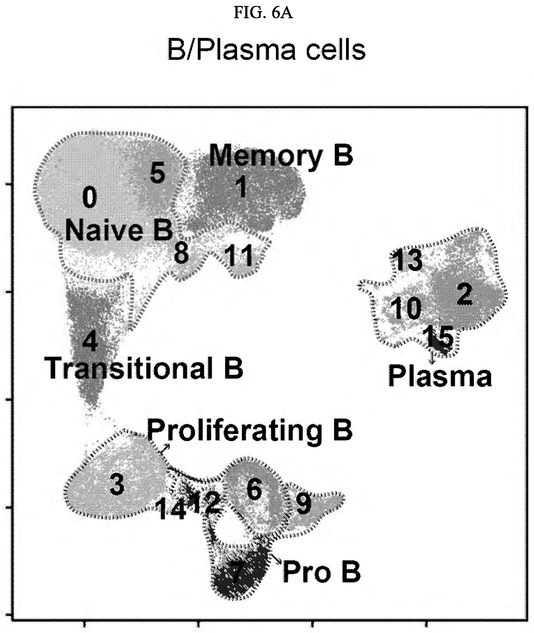

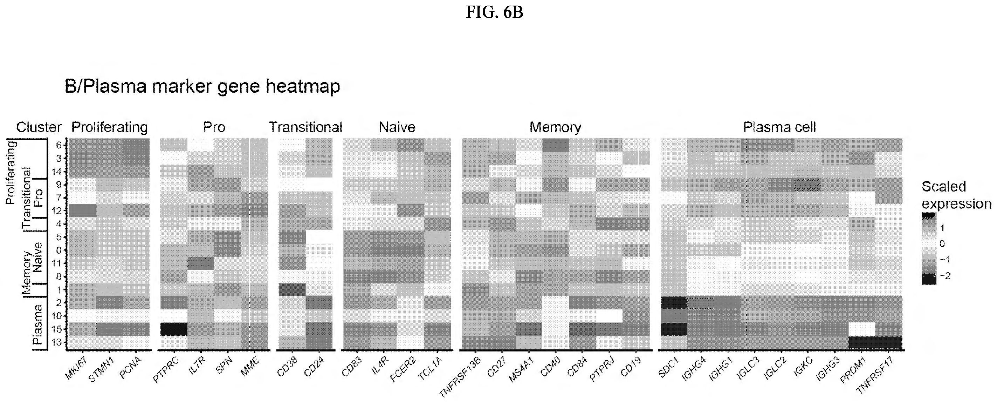

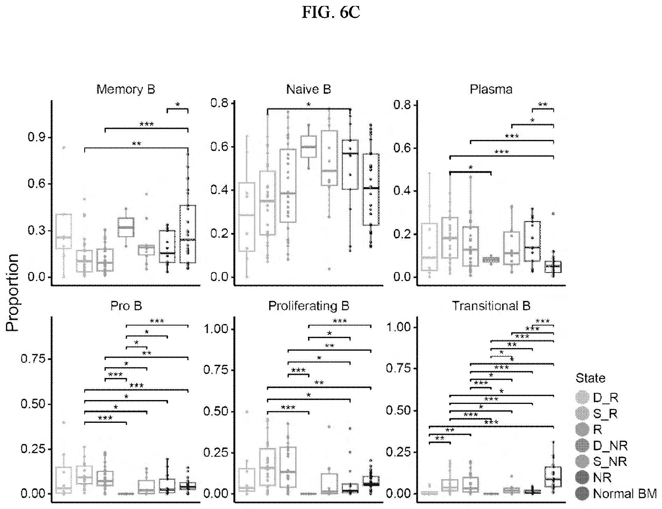

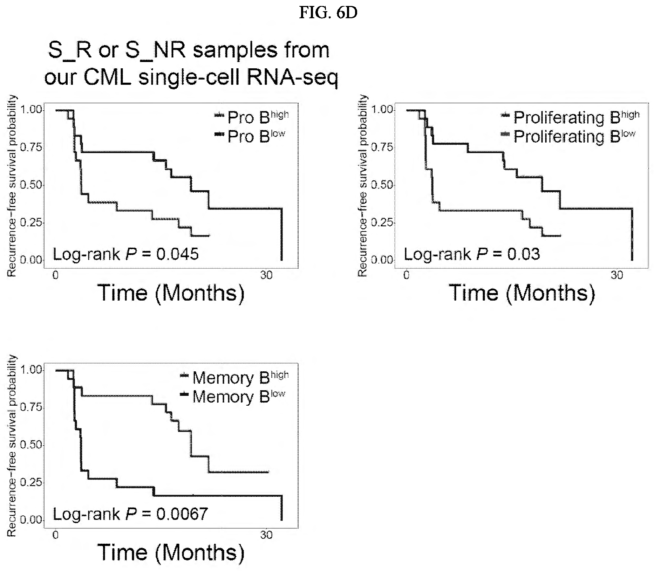

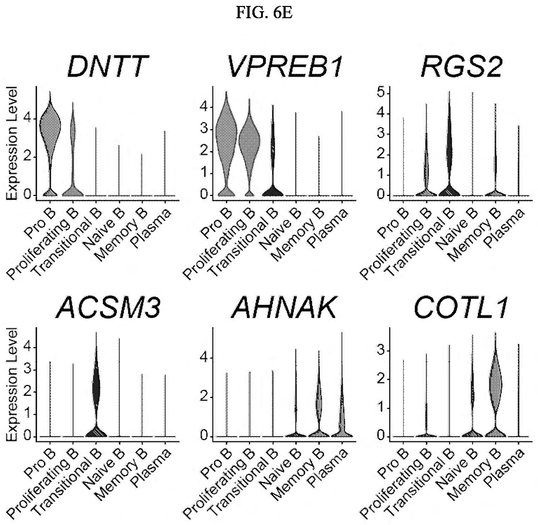

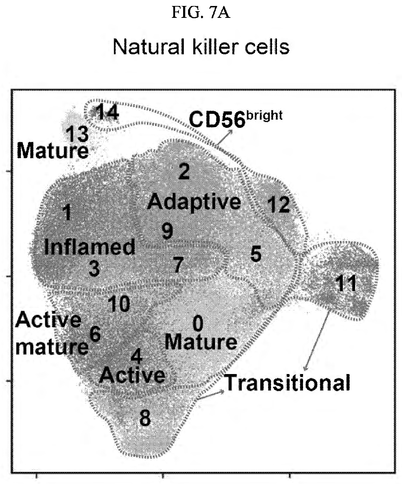

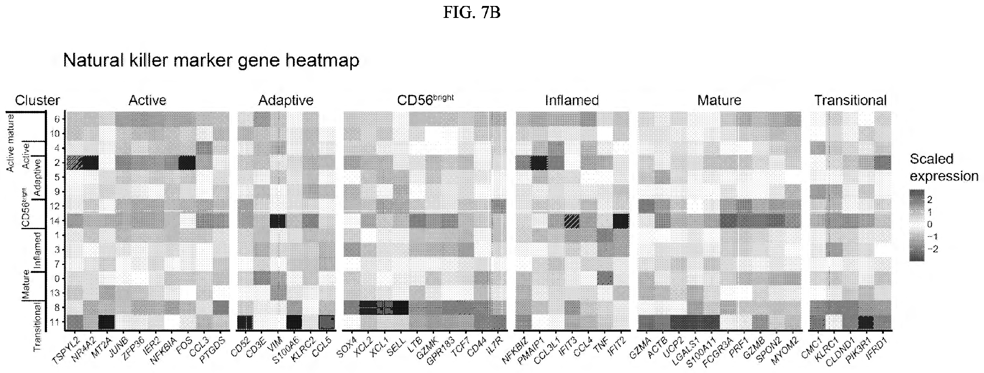

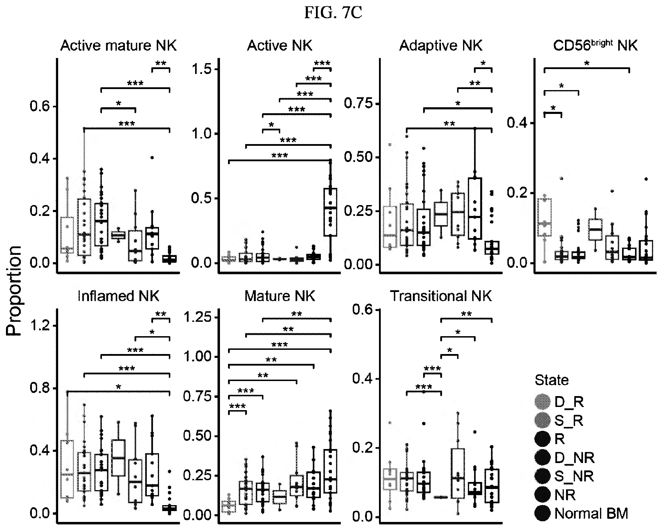

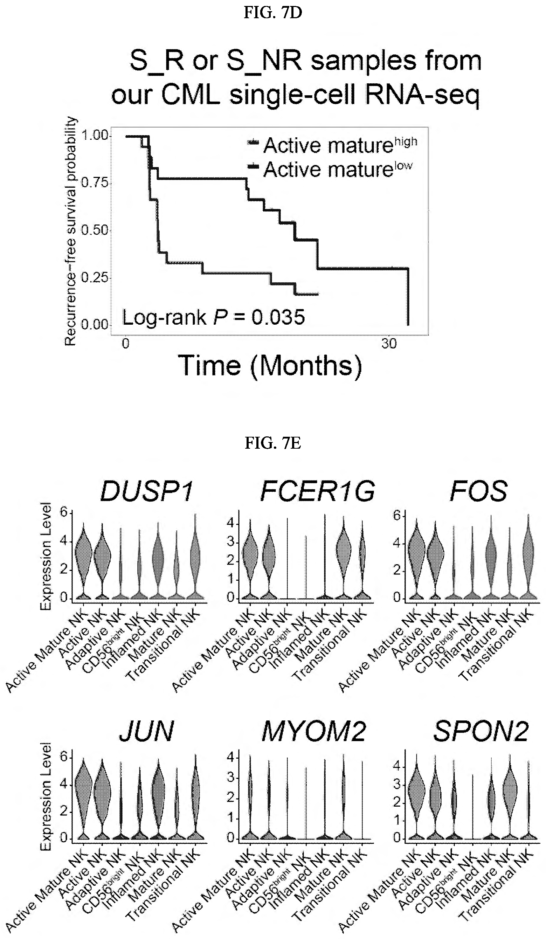

Subclustering analysis of B cells and plasma cells revealed numerous B-cell subsets, including proliferating B cells, pro-B cells, transitional B cells, Naive B cells, memory B cells, and plasma cells (FIGs. 6A and 6B). The proportion of each subset per sample was measured and compared with each other, including normal bone marrow. As a result, memory B cells were significantly abundant in normal bone marrow, whereas plasma cells were highly abundant in CML patients at the time point of TKI discontinuation and at the time point of relapse monitoring (FIG. 6C). Additionally, transitional B cells were exhausted in non-relapsing CML patients, whereas pro-B cells and proliferating B cells were significantly abundant in the relapsing patient groups (S_R and R). In the cohort, a higher proportion of pro-B cells and proliferating B cells in samples at the time point of TKI discontinuation was associated with a lower RFS (FIG. 6D). Conversely, patients with a higher memory B-cell proportion exhibited a better RFS (Log-rank P = 0.0067). It was found that pro-B cells and proliferating B cells highly expressed DNTT and VPREB1, which are genes commonly expressed in B-cell malignancies (FIG. 6E) (Mehtonen, J. et al., Genome Med. 12(1): 99. 2020). RGS2 and ACSM3 were highly expressed in transitional B cells. RGS2 is known as a mediator of cancer cell dormancy and tumor recurrence in non-small cell lung cancer, and ACSM3 was highly expressed in recurrent B-cell acute lymphoblastic leukemia. Furthermore, memory B cells highly expressed AHNAK and COTL1, which are known as tumor suppressor genes. Taken together, these results indicate that, in contrast to the abundance of memory B cells in healthy bone marrow, pro-B cells and proliferating B cells are abundant in relapsing CML patients at the time of TKI discontinuation and relapse. A higher proportion of NK cells showing upregulation of activation markers with a mature phenotype at the time of TKI discontinuation was associated with lower recurrence-free survival in CML patients. Considering that the abundance of cytotoxic NK cells at the time of TKI discontinuation is associated with better recurrence-free survival, de-novo clustering analysis was performed on NK cells to identify 7 major subsets: active NK, adaptive NK, CD56^{bright} NK, inflamed NK, mature NK, transitional NK, and active mature NK cells (FIGs. 7A and 7B). Active NK cells were significantly abundant in normal bone marrow, whereas inflamed NK cells were abundant in CML patients (FIG. 7C). Additionally, active NK cells with a mature phenotype (defined as "active mature NK cells") were significantly abundant in the relapsing patient groups (S_R and R) compared to normal bone marrow. Patients with a higher proportion of active mature NK cells at the time of TKI discontinuation exhibited lower RFS (Log-rank P = 0.035) (FIG. 7D). Active mature NK cells showed high expression of immediate early genes, including DUSP1, FOS, and JUN, similar to the active NK phenotype in human bone marrow and blood samples (FIG. 7E) (Yang, C. et al., Nat. Commun. 10(1): 3931, 2019). Furthermore, increased expression of genes associated with cytotoxic activity, including FCER1G, MYOM2, and SPON2, was observed in active mature NK cells (Cai, Y. et al., EBioMedicine. 53: 102686, 2020).

### Example 6: Unsupervised Hierarchical Clustering Analysis

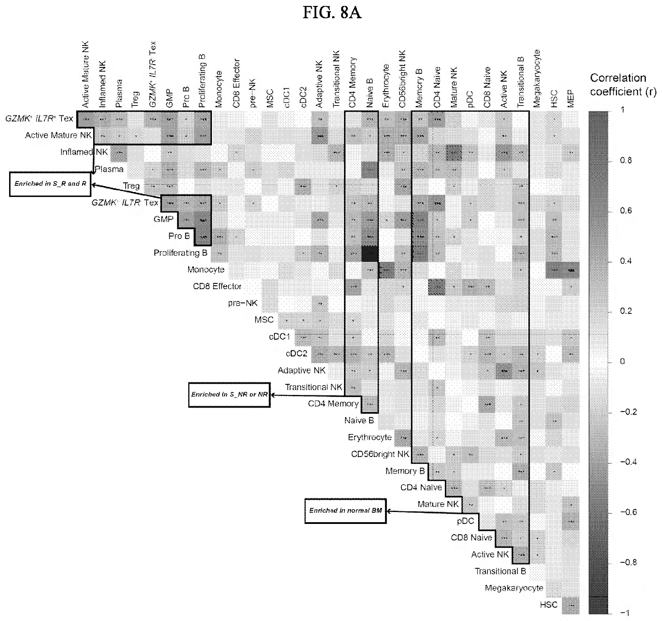

Intercellular correlations based on subset abundances in the bone marrow microenvironment and machine learning-based predictive analysis of relapse in CML patients were performed. To investigate how the various subsets of each major cell type are related to each other, unsupervised hierarchical clustering was performed using the individual frequency of subsets in each sample. Three distinct cellular clusters characterized by specific subsets enriched at particular time points in CML or normal bone marrow were identified (FIG. 8A). One cluster consisted of subsets enriched in the CML of relapsing patient samples (S_R and R) (AZU1⁺ GMP, GZMK⁺ IL7R⁻ Tₑₓ, pro-B cells, GZMK⁺ IL7R⁺ Tₑₓ, and active mature NK), which exhibited a positive correlation with each other. Another cluster consisted of subsets enriched in the CML of non-relapsing patient samples (S_NR or NR), CD4 memory and naïve B), which primarily showed a negative correlation with the subsets enriched in the relapsing patient samples (S_R and R). The other cluster consisted of subsets enriched in normal bone marrow (memory B, CD4 naïve, mature NK, pDC, CD8 naïve, active NK, and transitional B).

Next, a CML relapse prediction model was constructed using the subset proportions of each sample based on a Random Forest algorithm. To predict relapse, a machine learning classifier was designed to automatically determine whether a patient would relapse after TKI discontinuation, utilizing the proportion of each subset within the major cell types of each sample and clinical information regarding the relapse status. The classifier was trained as a binary (relapse vs. non-relapse) classifier utilizing relapse and non-relapse samples at the time point of TKI discontinuation (S_R and S_NR) or relapsing and non-relapsing patient samples at the time point of relapse monitoring (R and NR). Notably, both classifiers demonstrated the highest balanced accuracy (BA) utilizing the proportion of GZMK⁺ IL7R⁻ Tₑₓ cells (87% at the time of TKI discontinuation, 82% at the time of relapse monitoring) (Tables 4 and 5).

Given that there are many other subsets enriched at specific time points in CML patients, it was hypothesized that relapse could be predicted more accurately utilizing a combination of proportions of different subsets. Remarkably, the relapse status could be predicted with a balanced accuracy of 87-94% utilizing a combination of GZMK⁺ IL7R⁻ Tₑₓ and GZMK⁺ IL7R⁺ Tₑₓ cells in the case of the TKI discontinuation time point (S_R and S_NR), and a combination of GZMK⁺ IL7R⁻ Tₑₓ and CD8 naive T cells in the case of the relapse monitoring time point (R and NR) (Tables 6 and 7).

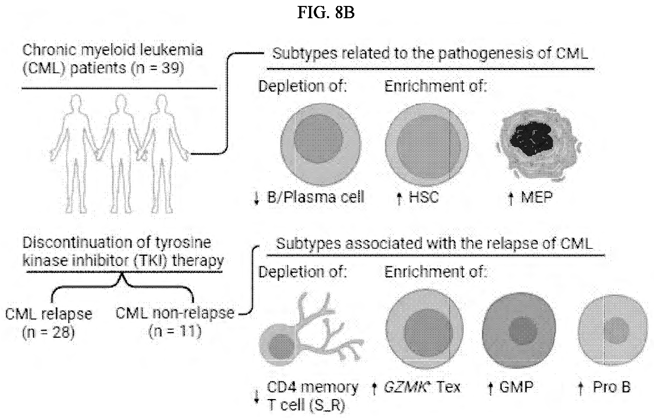

These results demonstrate the correlation of subtypes associated with the pathogenesis of CML or subtypes associated with the relapse or non-relapse of CML after TKI discontinuation, along with a machine learning-based classifier for predicting the relapse of CML after TKI discontinuation (FIG. 8B). The balanced accuracy (BA) scores of the binary Random Forest classifier utilizing the proportion of each cellular subset at the time point of TKI discontinuation or at the time point of relapse monitoring are summarized in Tables 4 and 5 below. The BA scores were sorted in ascending order as the average of sensitivity and specificity to handle imbalanced datasets.

**Table 4**

| Classification: S_R vs. S_NR | |
|---|---|
| Subtype | BA |
| cDC1 | 0.34 |
| pre-NK | 0.36 |
| CD8 effector | 0.38 |
| *GZMK⁺ IL7R*⁺ Tₑₓ | 0.38 |
| memory B | 0.41 |
| CD4 naïve | 0.43 |
| MSC | 0.44 |
| active mature NK | 0.44 |
| pDC | 0.45 |
| mature NK | 0.48 |
| transitional NK | 0.48 |
| CD56bright NK | 0.49 |
| inflamed NK | 0.5 |
| Naive B | 0.5 |
| monocyte | 0.52 |
| active NK | 0.52 |
| adaptive NK | 0.52 |
| MEP | 0.54 |
| plasma | 0.55 |
| HSC | 0.56 |
| cDC2 | 0.56 |
| GMP | 0.56 |
| CD4 memory | 0.56 |
| erythrocyte | 0.59 |
| T_{reg} | 0.59 |
| CD8 naïve | 0.61 |
| transitional B | 0.61 |
| proliferating B | 0.65 |
| Megakaryocyte | 0.67 |
| Pro B | 0.8 |
| *GZMK⁺ IL7R⁻* Tₑₓ | 0.87 |

**Table 5**

| Classification: R vs. NR | |
|---|---|
| Subtype | BA |
| adaptive NK | 0.34 |
| HSC | 0.35 |
| pre-NK | 0.38 |
| mature NK | 0.38 |
| plasma | 0.42 |
| proliferating B | 0.42 |
| Megakaryocyte | 0.43 |
| Erythrocyte | 0.44 |
| CD8 effector | 0.44 |
| Naïve B | 0.45 |
| Inflamed NK | 0.46 |
| active NK | 0.48 |
| MEP | 0.49 |
| monocyte | 0.5 |
| CD4 naïve | 0.5 |
| *GZMK⁺ IL7R*⁺ Tₑₓ | 0.51 |
| Pro B | 0.52 |
| transitional B | 0.53 |
| cDC1 | 0.54 |
| cDC2 | 0.54 |
| transitional NK | 0.59 |
| CD8 naïve | 0.6 |
| Memory B | 0.6 |
| MSC | 0.61 |
| pDC | 0.62 |
| T_{reg} | 0.63 |
| CD56^{bright} NK | 0.64 |
| Active Mature NK | 0.65 |
| CD4 memory | 0.66 |
| GMP | 0.69 |
| *GZMK⁺ IL7R*⁻ Tₑₓ | 0.82 |

Additionally, the BA scores of the binary Random Forest classifier utilizing the combination of proportions of cellular subsets in samples at the time point of TKI discontinuation or at the time point of relapse or non-relapse monitoring are summarized in Tables 6 and 7 below.

**Table 6**

| Classification: S_R vs. S_NR | | | | |
|---|---|---|---|---|
| Optimal Combination | BA | AUC | Sensitivity | Specificity |
| *GZMK⁺ IL7R⁻* Tₑₓ + *GZMK⁺ IL7R⁺* Tₑₓ | 0.94 (0.8-1) | 0.91 (0.67-1) | 0.88 (0.61-1) | 1 (1-1) |

**Table 7**

| Classification: R vs. NR | | | | |
|---|---|---|---|---|
| Optimal Combination | BA | AUC | Sensitivity | Specificity |
| *GZMK⁺ IL7R⁻* Tₑₓ + CD8 naïve | 0.87 (0.64-1) | 0.87 (0.78-0.97) | 0.89 (0.61-1) | 0.85 (0.52-1) |

While the present invention has been described with reference to the preferred embodiments thereof, it will be understood by those skilled in the art that various changes, modifications, and equivalent embodiments may be made therein without departing from the spirit and scope of the invention. Therefore, the true technical scope of protection of the present invention should be defined solely by the technical spirit and scope of the appended claims.

## Claims

1. A computer-implemented system for predicting molecular relapse in a patient having chronic myeloid leukemia (CML) undergoing a tyrosine kinase inhibitor (TKI) discontinuation protocol, the system comprising:
a biomarker data input interface configured to receive an immunological data vector generated from a biological sample of a CML patient at a TKI discontinuation milestone or a subsequent relapse monitoring milestone, wherein said data vector comprises a measured proportion of a first exhausted T-cell (Tₑₓ) subpopulation characterized as GZMK⁺ IL7R⁻ Tₑₓ cells and a measured proportion of a second Tₑₓ subpopulation characterized as GZMK⁺ IL7R⁺ Tₑₓ cells, each relative to total CD8⁺ T cells isolated from the biological sample;
a machine learning pipeline processor communicatively coupled to the interface and containing a trained machine learning classification network built on a machine learning algorithm, wherein the pipeline processor is configured to execute automated processing of the data vector by evaluating a numerical equilibrium between the proportion of the GZMK⁺ IL7R⁻ exhausted T cells (Tₑₓ cells) s and the proportion of the GZMK⁺ IL7R⁺ Tₑₓ cells to calculate a long-term molecular relapse probability score and classify the patient into either a high molecular relapse risk state or a stable treatment-free remission (TFR) state; and
a therapeutic intervention interface configured to generate and electronically display an interactive clinical dashboard partitioning the patient management profile into a target clinical roadmap according to the classification outputted by the pipeline processor,
wherein the dashboard outputs a specific digital instruction that directs a clinical practitioner to maintain the TKI discontinuation protocol without drug administration when the patient is classified into the stable TFR state, or to execute a predictive frontline TKI resumption regimen when the patient is classified into the high molecular relapse risk state.

2. The computer-implemented system according to claim 1, wherein the machine learning algorithm is a binary random forest (RF) algorithm or logistic regression (LR) algorithm.

3. The computer-implemented system according to claim 1 or 2, wherein the proportion of a first Tₑₓ subpopulation and the proportion of a second Tₑₓ subpopulation is measured by fluorescence activated cell sorter (FACS), single cell RNA sequencing, and/or single cell TCR sequencing.

4. The computer-implemented system according to any one claim among claims 1 to 3, wherein the biological sample is bone marrow sample or peripheral blood sample.

5. The computer-implemented system according to claim 4, wherein the bone marrow sample is longitudinal bone marrow sample.

6. A composition for detecting a specific biomarker combination in a bone marrow or peripheral blood samples obtained from chronic myeloid leukemia (CML) patient undergoing a tyrosine kinase inhibitor (TKI) discontinuation protocol comprising reagents for detecting the specific biomarker combination, wherein the specific biomarker combination comprises a proportion of GZMK⁺ IL7R⁻ exhausted T (Tₑₓ) cells and a proportion of GZMK⁺ IL7R⁺ Tₑₓ cells relative to total CD8⁺ T cells, for use in a method of managing a CML patient to predict molecular relapse after TKI discontinuation, wherein the method comprises:
measuring said proportion of GZMK⁺ IL7R⁻ Tₑₓ cells and said proportion of GZMK⁺ IL7R⁺ Tₑₓ cells in the biological sample obtained from the patient;
determining a molecular relapse risk state of the patient based on the measured specific biomarker combination, wherein an enrichment of the GZMK⁺ IL7R⁻ Tₑₓ cells relative to the GZMK+ IL7R+ Tex cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
identifying a therapeutic intervention for the patient, wherein a frontline TKI administration protocol is resumed or the prescription is switched to a 3^{rd} or 4^{th} generation TKI or combination therapy of an adjuvant or an immune check point inhibitor with the TKI when the patient is determined to be in the high molecular relapse risk state, and the TKI discontinuation protocol is maintained without drug administration when the patient is determined to be in the stable TFR state.

7. The composition for use according to claim 6, wherein the molecular relapse risk state of the patient is determined using a machine learning system

8. The composition for use according to claim 7, wherein the machine learning system comprises a logistic regression algorithm or a random forest algorithm.

9. The composition for use according to any one claim among claims 6 to 8, wherein the biological sample is bone marrow sample or peripheral blood sample.

10. The composition for use according to claim 9, wherein the bone marrow sample is longitudinal bone marrow sample.

11. The composition for use according to any one claim among claims 6 to 10, wherein the reagent may comprise:
i) an antibody set comprising antibodies against GZMK, antibodies against IL7R and antibodies against CD8;
ii) reagents for single cell RNA sequencing; or
iii) reagents for single cell TCR sequencing.

12. The composition for use according to claim 11, wherein the antibody set comprises functional fragments of the antibodies or antibody mimetics.

13. The composition for use according to claim 12, wherein the antibody mimetics is VLR, nanobody, V_{H}H, repebody, or monobody.

14. An *ex vivo* method of managing a CML patient to predict molecular relapse after TKI discontinuation, wherein the method comprises:
measuring the proportion of GZMK⁺ IL7R⁻ exhausted T (Tₑₓ) cells and the proportion of GZMK⁺ IL7R⁺ Tₑₓ cells relative to total CD8⁺ T cells in a biological sample that has been obtained from a patient undergoing a tyrosine kinase inhibitor (TKI) discontinuation protocol;
determining a molecular relapse risk state of the patient based on said measured proportions, wherein an enrichment of the GZMK⁺ IL7R⁻ Tₑₓ cells relative to the GZMK⁺ IL7R⁺ Tₑₓ cells determines a high molecular relapse risk state, and wherein an enrichment of the GZMK⁺ IL7R⁺ Tₑₓ cells relative to the GZMK⁺ IL7R⁻ Tₑₓ cells determines a stable treatment-free remission (TFR) state; and
identifying a therapeutic intervention for the patient, wherein a frontline TKI administration protocol is selected for resumption or the prescription is switched to a 3^{rd} or 4^{th} generation TKI or combination therapy of an adjuvant or an immune check point inhibitor with the TKI when the patient is determined to be in the high molecular relapse risk state, and/or the TKI discontinuation protocol is maintained without drug administration when the patient is determined to be in the stable TFR state.

15. The *ex vivo* method of claim 14, wherein the biological sample is bone marrow sample or peripheral blood sample.
